# EUROPEAN PATENT SPECIFICATION

(11) **EP 3 636 231 B1**
(45) Date of publication and mention of the grant of the patent: **24.04.2024**
(21) Application number: 18200099.2
(22) Date of filing: 12.10.2018
(51) Int. Cl.: A61F 13/00, A61F 13/08

(54) **MEASUREMENT DEVICE, COMPRESSION DEVICE AND METHODS RELATED THERETO**
MESSVORRICHTUNG, KOMPRESSIONSVORRICHTUNG UND ZUGEHÖRIGE VERFAHREN
DISPOSITIF DE MESURE, DISPOSITIF DE COMPRESSION ET PROCÉDÉS ASSOCIÉS

(43) Date of publication of application: 15.04.2020
(73) Proprietor: Medi USA, L.P., Whitsett, NC 27377 (US)
(72) Inventor: LIPSHAW, Moses, Hillsborough, North Carolina 27278 (US)
(74) Representative: Lindner Blaumeier Patent- und Rechtsanwälte

(56) References cited:
- EP-A1- 2 220 996
- DE-A1-102004 022 504
- US-A1- 2003 135 146
- US-A1- 2011 009 795

## Description

The invention concerns a measurement device for measuring a tension and/or a displacement of a textile compression material of a compression device or a compression force applied to a patient and/or a displacement of the compression device, a compression device and methods related thereto.

Compression devices used for compression therapy of a limb of a patient are already known in the state of the art. For example, compression wraps, compression stockings, compression bandages and other devices have been proposed and used. Such compression devices, in particular wearable and non-wearable devices, often include closure systems that are reliant on the user to determine the appropriate level of tension for device performance and/or personal comfort. For example, the compression device may comprise a body portion to be wrapped around the limb with bands extending therefrom whose free ends may be attached to the body portion and/or the bands itself. Depending on how tight the bands are wrapped around the body portion and thus, the limb, various compression levels may be applied to the limb. Usually, compression profiles to be applied to the limb are determined depending on the object of the therapy.

The successful application of compression devices is based on training, prior experience and/or trial and error. Thus, in particular for compression devices and applications where compression is critical, expensive traditional tension measuring devices (tensiometers), for example spring scales or electronic strain gauges have been proposed. The adaptation of traditional tensiometers for compression garments is often cost prohibitive and the integration of the hardware is either not viable or not desired.

Compression devices, in particular compression therapy devices, have for example been proposed for the treatment of circulatory disorders such as lymphedema and venous disease. However, this disclosure is not limited to such compression devices, but can also be applied to other compression devices, in particular wearable compression devices.

Regarding wearable compression devices, it has been proposed in the state of the art to use so-called Built-In Pressure & Tension Systems, wherein the textile compression material is marked with indicia. As the textile compression material is tensioned, it elongates and the indicia spread further apart. The corresponding tension measurement devices now include an external scale card comprising a scale which associates a measured elongation using the indicia with a tensionbased output. However, these tension measurement devices rely on consistent stretch and recovery properties of the textile compression material used which may have variances between production lots and over use life. Therefore, the accuracy of the tension measurement device may be limited. Additionally, this design requires a separate scale to measure the elongation. There is no instant communication of the current tension/compression to the user. While it may be possible to fix the scale card or scale in general to the exterior of the compression device in a two-layer type system to communicate tension and thus compression levels, the tension measurement system is still limited to measuring elongation, not tension, and is dependent on the performance and properties of the textile compression material.

In another approach, in particular for compression devices like bandages, it has been proposed to incorporate printed designs onto the textile compression material such that as the textile compression material elongates, the printed design geometry changes and forms a noticeable shape to communicate the appropriate tension has been achieved. Such compression measurement systems are also susceptible to the stretch and recovery material property variance which may limit accuracy. Furthermore, observing the geometry change is subjective and can vary between users resulting in different applied tensions and thus compressions.

A primary deficiency of these prior art solutions is that they do not directly measure force, in particular tension force, but indirectly calculate an applied tension force using a secondary measure.

Magnetic fastening systems are disclosed in US 2011/0009795 A1, DE 10 2004 022 504 A1, and US 2003/0135146 A1. US 2011/0009795 A1 provides a hybrid compression garment combining the advantages of static and active compression garments, wherein magnets are mentioned as possible connectors. DE 10 2004 022 504 A1 addresses de-congestion of a tissue to be treated, wherein magnetic strips are mentioned in a list of fastening means. US 2003/0135146 A1 relates to resolving problems when attaching wound dressings using adhesives and mentions magnetic means in a list of attachment means.

EP 2 220 996 A1 concerns a pressure indicator for medical pressure bandages (tourniquets). In a device for monitoring whether the correct pressure has been reached, a sensor unit and an evaluation unit communicate wireless by inductive connection. The sensor unit is comprised by a first coil, which can be shortcircuited by a mechanical pressure switch, and the evaluation unit comprises a resonator circuit having a second coil.

It is thus an object of the current invention to provide a simple, durable, and cost-effective measurement device to measure, adjust and instantly communicate tension levels.

This object is achieved by providing a measurement device, a compression device, a measuring method and a method for designing a measurement device or a compression garment with at least one measurement device according to the independent claims. Advantageous embodiments are described by the dependent claims.

A measurement device for measuring a tension and/ or a displacement of a compression material of a compression device or a force and/or a displacement associated with the compression device applied to a patient therefore comprises at least one first and at least one second magnetic element, wherein the first and the second magnetic elements are relatively displaceable to each other such that the tension and/or force is measurable depending on a relative displacement of the first and second magnetic elements to each other based on a tension and/or force overcoming a magnetic interaction force between the first and second magnetic elements. The measurement device further comprises a guiding element to guide the relative displacement of the first and second magnetic elements. Preferably, the first and/or second magnetic elements are magnetizable or permanently magnetic.

Magnets (e.g. ferromagnetism, paramagnetism, diamagnetism,
and antiferromagnetism) and/or ferromagnetic materials exert an attractive magnetic interaction force onto each other. It is already known that these magnetic interaction forces vary according to material properties, size, dimension, geometry, axis of polarity, and a contact area, while relative spatial positioning of magnetic elements and/or distances between materials also influence the strength of magnetic interaction forces. The current invention, in preferred embodiments, proposes a magnetic tensiometer design where the change in spatial positioning of the paired magnetic elements due to tension forces applied by a user overcoming at least a portion of the magnetic interaction force communicates that a specific tension force has been achieved, such that predefined compression levels may be selected and applied by the compression device. The first and the second magnetic elements are arranged, in particular, in a base position corresponding to a base tension state, which is preferably a non-stretched state, such that they are displaceable relative to each other due to tension forces applied by a user. If this tension force overcomes the magnetic interaction force or at least a portion thereof, a relative displacement between the first and second magnetic elements occurs which indicates the strength of the tension force to the user.

Please note that if the tension applied by the user and preferably measured by the measurement device according to the invention, which may also, in the case that it is a tension measurement device, be termed tensiometer, refers to non-stretched textile compression material, the tension level directly relates to a compression level applied by the so-stretched textile compression material.

An advantage of the current invention is that a tension force may be directly measured. That is, while textile power may deteriorate over time, the magnetic interaction force used as a reference in the measurement device does not, at least not essentially, when both effects are compared. As long as a user is able to apply the appropriate tension force and the closures of the compression device remain intact, the durability and accuracy of the tension and compression is maintained. In summary, the measurement device is of a simple and cost-effective design and may instantly communicate tension levels or other measurement results. It is durable and allows to reliably apply the determined tension forces and thus compression levels.

While this disclosure may be focused on designs particularly suitable for wearable compression therapy devices, in particular compression garments for circulatory disorders such as lymphedema and venous diseases, it is to be understood that the potential application of the measurement device extends beyond the field of wearable compression devices.

The measurement device according to the invention is preferably applied to closure systems of compression devices, for example bands extending from a body portion and wrapped around the body portion with a tension force such that a desired compression is applied by the textile compression material, of which the bands as well as the body portion may be made. It is noted that if only one of the first and second magnetic elements is directly or indirectly attached to the textile compression material, the other magnetic element should be placed in an affixed position not influenced by other tension forces. It is, however, preferred to have both magnetic elements coupled to the textile compression material, as will be explained below in numerous examples.

At least one of the magnetic elements is preferably ferromagnetic, but it is also conceivable to induce magnetism into a magnet by using electric current, for example a coil, such that the at least one magnetic element is magnetizable (and magnetized when used during the measurement). An advantage of using a nonpermanent magnet is that different currents, leading to different field strengths and therefore different magnetic interaction forces, may be used. To provide different currents to magnetize a magnetic element, a power source and/or a controller may be provided. It is also noted that, while it suffices to have one magnetic element permanently magnetic or magnetizable, preferably both magnetic elements are ferromagnetic and permanently magnetized, or magnetizable. It has been shown in experiments conducted by the inventors that even magnets of small size, having suitable properties influencing the magnetic interaction force, attract each other with an interaction force allowing measurement of tension forces usually applied in compression devices, in particular in closure systems while finalizing the donning of compression device, and, consequently, associated compression levels.

In preferred embodiments, the measurement device, in particular the first and/or the second magnetic elements, may be attachable to the compression device by fastening means. That is, at least a part of the measurement device, in particular at least one of the first and/or second magnetic elements, may be attached to the compression device, in particular the compression material, to be able to measure a tension, displacement and/or force. The fastening means may be configured to releasably or non-releasably attach the measurement device, in particular the first and/or second magnetic elements, to the compression device. A releasable attachment is advantageous in cases where elements of the measurement device are to be replaced and/or the measurement device is configured to be completely removable due to design and/or usability considerations. Preferably, the fastening means may comprise a hook-and-loop fastener and/or a magnetic fastener. In particular, regarding the magnetic fastener, at least one of the at least one first and/or at least one second magnetic elements may form part of such a magnetic fastener. In concrete embodiments, a hook material engaging the textile compression material or loops on the textile compression material or vice versa may be used.

The measurement device may comprise audible and/or visual and/electronic and/or sensory indication means indicating the displacement of the at least one first and at least one second magnetic elements. To inform the user of the displacement of the pair of magnetic elements used for measurement, various indication means may be used. For example, visual indication means may include indicia becoming visible on displacement of the magnetic elements, while audible and/or electronic indication means may, as well as in some cases visual and/or sensory indication means, be powered by an electric power source, for example a battery and/or a generation means, in particular using energy generated by the displacement itself to power indication means. For example, in compression devices which, when applied, do not provide unrestricted view onto the compression device, audible, electronic and/or sensory indication means may be preferred. Indication means may also be placed apart from the pair of magnetic elements that is displaced relatively to each other, for example if the displacement is communicated wirelessly to the indication means.

Generally speaking, the indication means may be connected directly or indirectly to the measurement device and/or the compression device. In some cases, the indication means may comprise a display showing measurement results, for example in terms of displacement distance and/or applied tension force and/or tension level. However, in concrete embodiments, it is preferred that the visual indication means comprises a scale indicating a range of displacement of the at least one first and/or at least one second magnetic element. The range of displacement may also be expressed in form of force and/or tension. In other embodiments, a translation table may be provided which associates tension force and/or tension levels with ranges of displacement indicated by the scale.

In numerous embodiments, it may be preferred to further define the allowable relative displacement of the pair of magnetic elements to tailor the measurement capabilities to the measurement needs associated with the compression device and to provide mechanical integrity of the measurement device itself. Thus, in preferred embodiments, the measurement device comprises at least one constraining element to limit the relative displacement of the at least one first and at least one second magnetic element. Such constraining elements may work in different ways, for example limiting how close the first and second magnetic elements may come to each other, how far they may be displaced from each other and define displacement orientations.

Therefore, at least one of the at least one constraining element may be a spacer to limit the relative displacement of the first and second magnetic elements to one another, in particular define an indirect contact position of closest distance which may, in embodiments, also be a base position. Alternatively or additionally, at least one of the at least one constraining element may be a limiter, for example a tether to restrict the relative displacement of the at least one first and at least one second magnetic elements away from each other. Finally, at least one of the at least one constraining element may be the guiding element to limit the relative displacement of the at least one first and at least one second magnetic element to at least one specific orientation. Concrete embodiments comprising one or more such constraining elements will be described in detail below. Constraining elements of the different types named above may also be used complementary or as combined elements. For example, embodiments which have a spacer, a limiter and the guiding element are conceivable as well as embodiments having one constraining element acting as a at least two of a limiter, a spacer and/or the guiding element.

In preferred embodiments, the measurement device configuration allows relative a displacement of the at least one first and at least one second magnetic element by continuous, intermittent direct, and/or intermittent indirect contact. In particular, multiple classes of embodiments of the measurement device according to the invention are conceivable. In a first class of embodiments, the pair of first and second magnetic elements is in continuous direct or indirect contact, for example, sliding relatively to each other. In a second class, the pair of first and second magnetic elements may be in direct contact in, for example, the base position, and are separated when a tension force overcoming the magnetic interaction force, for example, is applied. Regarding a third class, for example, spacers may be used to provide indirect contact in the base position and thus modify the magnetic interaction force to be overcome for separation. Concrete examples will be described below in more detail.

In preferred embodiments, the first and/or second magnetic elements, the indication means, the fastening means and/or the at least one constraining element are part of and/or protected by and/or fixed to and/or contained in and/or on a supporting element. Such a supporting element may, for example, be a holder, a cover or the like. The supporting element may establish mechanical cohesiveness of the measurement device or parts thereof.

In especially preferred embodiments, the measurement device comprises multiple first and/or second magnetic elements, constraining elements, fastening means, indication means and/or supporting elements, such that a desired magnetic interaction force strength and/or displacement is selectable. Providing multiple elements of the measurement device allows configuration of the measurement device to provide suitable measurement conditions. In particular, the multiple elements of the measurement device may be releasably combinable with each other to provide a modular measurement device. For example, if different first magnetic elements having different magnetic properties are chosen, different magnetic interaction forces result when combined with the same second magnetic element. In another example, different spacers as constraining elements may be used to define different base positions associated with different magnetic interaction forces to be overcome to separate the pair of magnetic elements spaced apart by the spacer. By appropriately combining elements of the measurement device to yield a configuration having at least one first and at least one second magnetic element, the measurement device can be adapted to suit the needs of particular measurements, for example, to measure whether a certain compression level of the compression device has been reached or the like.

The multiple elements of the measurement device, in particular the multiple first and/or second magnetic elements, constraining elements, fastening means, indication means and/or supporting elements, may preferably comprise different properties, in particular material composition, geometry, and/or dimension. That is, the multiple elements may have different properties regarding the magnetic interaction force, which may also be termed Magnetic Design Properties (MDP). If multiple elements having different magnetic design properties exist in a modular measurement device, these can be combined to yield defined magnetic interaction forces and thus defined measurement properties.

In concrete embodiments, the multiple first and/or second magnetic elements may be arranged in series to each other or be optionally combinable with each other such that the pairs of magnetic elements successively indicate the respective defined forces and/or displacements with change of strength of the force applied. Multiple pairs of first and second magnetic elements may be used in the same measurement device, each pair corresponding to a defined desired magnetic interaction force strength and/or displacement. If, for example, these pairs of magnetic elements are in direct or indirect contact in a base position and indicate tension forces corresponding to the respective defined magnetic interaction force strength by being separated, application of an increasing tension force leads to the multiple pairs of first and second magnetic elements successively separating. Of course, other embodiments having a different configuration to measure tension, force and/or displacement are also conceivable.

Preferably the measurement device may comprise indicia, in particular relating to the compression device such that the respective measurement device or measurement or measurement device configuration is selectable. In concrete embodiments, the multiple elements each may have indicia allowing to discern corresponding defined magnetic interaction force strength and/or displacements and/or compression devices for which the elements are suitable. In other embodiments, if multiple measurement devices are available, the measurement device itself may be characterized by the indicia such that a measurement device suitable for a defined compression device or location on a compression device may be selected. The indicia may be provided in numerous ways, for example by integrating text and/or symbols into textile compression material and/or another material of the measurement device or the multiple elements, respectively, by using patches, different colors and the like.

A compression device according to the invention comprises at least one compression material, in particular a textile compression material, and at least one measurement device according to the invention, in particular to adjust the compression device according to a predetermined compression profile of the compression device. The compression profile may be provided, in particular, in a known standard, for example, RAL, US standard or the like. Preferably, the measurement device may thus be used to adjust the compression device to apply the predetermined compression profile to the patient, for example the limb of the patient. The measurement device may, for example, indicate that, in particular locally, a certain compression level according to the compression profile has been achieved. It is noted that all features and remarks regarding the measurement device according to the invention also apply to the compression device according to the invention.

Preferably, the measurement device, in particular at least one of the magnetic elements, may at least partly be integrated into the compression device, or vice versa. In this manner, the measurement device may form an integral part of the compression device, in particular always being available when needed for a tension/compression measurement. For example, magnetic elements may be placed in pockets inside the bands or the body portion of a compression device.

Preferably, the compression device comprises at least one band for adjusting the compression of the compression device, wherein the measurement device is at least partly integrated into or applied to at least one of the at least one band. In particular, the compression device may comprise a body portion that wraps around the limb and at least one band extending from the body portion that wraps around the body portion and can be attached onto the body portion or one of the at least one bands. Using the at least one band, a textile compression material of the compression device may be stretched due to a tension force applied to the band which may be measured using the measuring device. As soon as a tension force corresponding to a compression level to be applied is indicated by the tension measurement device, the band may be attached to the body portion and/or one of the at least one band to maintain the stretching such that the textile compression material applies the corresponding compression to the limb.

In a particularly advantageous embodiment, the compression device may comprise multiple bands, at least two of the multiple bands each having an associated measurement device. That is, multiple tensiometers could be used to adjust multiple bands upon application. In particular, different pairs of first and second magnetic elements resulting in different magnetic interaction forces may be used for the tension measurement devices of at least two different bands, in particular chosen according to a predetermined compression profile for the compression device. In this manner, different compression ranges/levels along the limb/extremity can be achieved.

Generally speaking, in preferred embodiments, multiple measurement devices may be associated with different locations on the compression device, wherein at least two of them have different measurement configurations, in particular chosen according to the predetermined compression profile of the compression device. For example, knowing the user's circumference at various points of the limb covered by bands, magnetic interaction forces could be selected resulting in particular in a certain defined tension force and thus allowing to adjust the band to provide predetermined compression to the section of the limb covered by this band. Compression profile standards such as RAL or US Standard could be achieved along with any other profile desired. If the compression device, in particular the compression garment, is further marked with indicia to measure the user's circumference as the compression device is applied, the user or manufacturer could easily reference a force-circumference-compression matrix to select the appropriate magnetic design properties of first and second magnetic elements and thus tensiometer strengths of the compression measurement devices for each area of the compression device. Preset configurations regarding tension measurement devices could also be communicated in the instruction manual for standard-size, "off-the-shelf" compression devices, in particular compression garments, to change the compression levels and/or profile.

There may be cases in which measurement with only one measurement device is sufficient to achieve a predetermined compression profile, in particular if the compression profile of the compression device is controlled by features of the compression device and/or the limb to which it is applied, preferably only leaving one degree of freedom. This is, for example, the case for a compression wrap on a conical leg providing a defined compression gradient.

It is noted that measurement devices that may be attached to ends of the compression device can also be considered for one-time use. For example, during a first application of the compression device, measurement devices may be attached to each of the bands to adjust these bands regarding tension during the first application of the compression device. The location of the band ends, i.e. the position where they are reattached onto, for example, the body portion and/or the same or another band, can then be marked on the compression device to repeat the application without using a tension measurement device. Further, the measurement devices may of course be re-used to create new markings over the course of use of the compression device to account for compression device changes and/or patient limb changes.

As has already been mentioned, the invention may be applied to all types of compression therapy devices using textile compression materials. In particular, the compression device may be or comprise a compression wrap and/or a compression sleeve and/or a compression bandage and/or a compression band and/or a compression garment and/or a compression stocking. In particular, the compression device may be or comprise wearable or non-wearable compression devices. The invention may also be applied to devices that do not entirely or partially consist of textile materials. For example, a compression device could consist of molded and/or flexible non-textile (e.g. metal, plastic) materials that also permit force measurement with the invention. The invention is also not limited to measuring force applied by the user but could also measure the applied force of another source (e.g. active smart textiles, memory alloys, mechanical system, electro-mechanical, pneumatics, phase change, etc.).

The invention also concerns a method for measuring tension and/or displacement of a compression material of a compression device or force and/or displacement associated with the compression device applied to a patient, wherein a measurement device comprises at least one first and at least one second magnetic element, wherein the first and the second magnetic elements are relatively displaced by an applied force and tension and/or displacement and/or force is measured depending on a relative displacement of the magnetic elements based on the tension force overcoming a magnetic interaction force between the first and second magnetic elements. All corresponding features and remarks regarding the measurement device and the compression device may also be applied to the measurement method according to the invention such that the same advantages can be achieved.

Finally, the invention also concerns a method for designing a measurement device or a compression garment with at least one measurement device according to the invention, comprising:
- providing at least one compression material wrapable at least partially around a body part to provide compression to the body part,
- determining a tension and/or compression value and/ or compression profile for the compression material,
- selecting at least one first and at least one second magnetic element according to a predetermined correlation between magnetic interaction forces and tension or compression values,
- assembling the at least two magnetic elements with the compression material to a measurement device or to a compression garment comprising the measurement device.

That is, once a selection of first and second magnetic elements having different properties is provided, measurement devices may be, so to say, specially tailored for the measurement tasks of certain compression devices and/or patients. For example, if a predetermined compression profile for a patient is prescribed, suitable magnetic elements and thus suitable magnetic interaction forces may be choses such that a user may reliably adapt the compression device during application to exert the predetermined compression profile, as the local measurement allows to determine whether the required tension and thus compression is present.

In a concrete embodiment of the design method, the step of selecting the at least two magnetic elements comprises:
- considering the circumference of the body part covered by the compression material and selecting the at least two magnetic elements according to a predetermined correlation between magnetic interaction forces, circumference of the body part and tension or compression values.

For example, of a predetermined compression profile is to be achieved, suitable tension or compression values may be choses according to the predetermined compression profile for each diameter, such that magnetic interaction forces defining the requirements for the magnetic elements can be taken from the correlation.

The measurement devices in such a design method, to which, of course, all remarks and features regarding the compression device, the measurement device, and the measurement method also apply, preferably indicates that a certain tension resulting in a desired compression level is reached when applying the compression device to the patient. For example, the pair of first and second magnetic element may be separated upon reaching the desired force, indicating this event to a user.

Generally speaking, in other words, particularly preferred embodiments of the invention relate to a tension measurement device for measuring a tension of a textile compression material of a compression device for a limb of a patient comprises at least one first and at least one second magnetic element, of which at least one is magnetizable or permanently magnetic and wherein at least one of the magnetic elements is attached or attachable to the textile material, wherein the first and second magnetic elements are relatively displaceable such that tension is measurable depending on a relative displacement of the magnetic elements based on a tension force overcoming a magnetic interaction force between the first and second magnetic elements. Advantageous concrete examples for such a configuration will now be described.

Preferably, the first and second magnetic elements are held in a base position corresponding to a base tension state, in particular a non-stretched state of the textile compression material, by the magnetic interaction force such that stretching of the textile material resulting in a tension force overcoming at least a defined portion of the magnetic interaction force changes the relative position from the base position to at least one second position to indicate the tension of the textile compression material. In particular, the textile compression material is not stretched when the first and second magnetic elements are attached in the base position, preferably directly or indirectly, for example using a spacer as the constraining element, contacting each other, in particular in a plane at least essentially perpendicular to the direction of the tension force (and thus in particular the plane in which the textile compression material extends). In this manner, when a tension force is applied, only the magnetic interaction force has to be overcome to effect relative displacement of the first and second magnetic elements. If the textile compression material had already been stretched, a return tension force, that is a compression force, would additionally be present which would also have to be overcome. In cases where the tension force only works against the magnetic interaction force and thus refers to a non-stretched state, the measured tension force corresponds to the resulting elastic compression force of the textile compression material and thus the compression level applied.

It is noted that, for some embodiments, the second position may be broadly defined, in particular for embodiments where (directly or indirectly) contacting first and second magnetic elements are separated once the magnetic interaction force is overcome by a defined tension force. In these embodiments, the base position would be contacting first and second magnetic elements, while the second position would be separated first and second magnetic elements.

However, in any case, it is preferred to use the at least one constraining element to limit the displacement of the first and second magnetic elements against each other when they disconnect, in particular such that when the applied force vanishes, they automatically reconnect to the base position due to the magnetic interaction force. Note that the constraining element may in some cases even be realized by the textile compression material.

In advantageous embodiments, the magnetic elements are releasably attachable, in particular directly or indirectly, to the textile compression material using a fastening means, wherein the fastening means allows the relative displacement of the first and second magnetic elements. This may be provided by allowing uninhibited stretching in at least a portion of the area of the textile compression material influencing the relative position of the first and second magnetic elements between or below these or by folding in particular non-elastic material up between the first and second magnetic elements in the base position, providing a certain base movement distance. For contacting or indirectly contacting first and second magnetic elements in a plane essentially perpendicular to the plane in which the tension force is exerted, a portion of an area of uninhibited stretching may comprise the plane in which the textile compression material extends below the contact area. In other words, the fastening means is not continuous along the length of the tension measurement device because a portion of the textile compression material must be free to elongate to allow the first and second magnetic elements to separate or, in general, be displaced relative to the other. In the case of a highly elastic textile compression material, the separation between fasteners of the two magnetic elements may be very low or the fastener separation edges may even be flush. In a very low stretch material a further separation of fasteners associated with each of the first and second magnetic elements may be necessary to provide enough room for displacement, in particular separation of the first and second magnetic elements.

In a concrete embodiment, the fastening means may comprise a hook-and-loop fastener, in particular a hook material engaging the textile compression material or loops on the textile compression material or vice versa. The fastening means may thus comprise hook-and-loop-fasteners for both the first and the second magnetic elements, wherein, if loops are provided on the textile compression material, hooks for at least one of the first and second magnetic elements may be omitted in an area of the textile compression material provided for elongation to allow the relative displacement of the first and second magnetic elements.

It is noted again that alternatively, the tension measurement device may also be at least partly integrated into the compression device, for example be sewn or otherwise permanently affixed to or inside the bands and/or a body portion of the compression device. In this manner, the tension measurement device forms an integral part of the compression device. It is, however, preferred to have at least one of the first and second magnetic elements releasably attached to the compression device to allow for replacement, in particular to adjust the magnetic interaction force by choosing a replaced magnetic element having different properties influencing a magnetic interaction force. Another advantage of releasable attachment via a fastening means is reduced assembly time and the possibility to "reset" the tension measurement device in case the textile compression material stretches out, as described below. Using releasable attachment, the compression measurement device may also be moved to other positions on a compression device for measurement.

The reliability of the tension measurement device is also advantageous regarding aging or migration of the textile compression material for several reasons. Firstly, the tension measurement device also provides a means to notify the user when the tension has dissipated due to garment migration or loss of power in the textile elasticity. For example, in the event that the textile compression material "stretches out", the first and second magnetic elements may become permanently separated. In such a case, the tension measurement device may be removed and re-attached. As the magnetic interaction force remains essentially constant over time, the re-attached tension measurement device again allows to stretch the (aged) textile compression material with a tension force necessary to apply the desired compression level.

In preferred embodiments, as already indicated, the first and second magnetic elements contact directly, or indirectly via a spacer, in the base position such that the exertion of a defined tension force corresponding to the magnetic interaction force results in indication by separation of the contacting magnetic elements. In particular, the contacting areas may be at least essentially perpendicular to a plane in which the textile material extends and in which the tension force is exerted. In the base position, into which the first and second magnetic elements have preferably been attached while the textile compression material was not stretched, the magnetic interaction force holds the first and second magnetic elements in contact. A magnetic interaction force is exerted essentially parallel to a direction in which a tension force will act when a user stretches the textile compression material of the compression device. While such a user-initiated tension force is lower than the magnetic interaction force, the contact between the first and second elements is maintained. As soon as the tension force surpasses the magnetic interaction force, the first and second magnetic elements are separated into the second position, thereby indicating haptically and visually that a tension corresponding to the magnetic interaction force (which is defined by design) has been reached. If the magnetic design properties of the first and second magnetic elements influencing the magnetic interaction force have been chosen so that a defined tension force corresponding to a compression level to be applied leads to separation of the magnetic elements, the moment, in which the tension force sufficient to result in the predetermined compression level is reached, is communicated directly to the user, who can stop tensioning the textile compression material and, for example, use a closure system of the compression device, in particular hook-and-loop-fasteners or latching fasteners, to finalize adjusting the compression. By decreasing the tension force slightly, the user may fine-tune the positioning, "feeling" the correct defined tension force by matching the magnetic interaction force as exactly as possible. Of course, in addition to the indication by separation itself, additions indication means, as described above, may also be used.

It is noted at this point, that, generally, a spacer is only an example for at least one constraining element which can be part of the tension measurement device. A spacer allows to adjust the magnetic interaction force. A spacer could also be used in other embodiments, for example to define a contact area when magnetic elements slide with respect to each other or to build a layered sliding system, as will also be described below.

Generally speaking, as already explained, the tension measurement device may comprise at least one displacement constraining element, such as a spacer, tether, guide, notch, slot, track, rotor, pocket, material, and/or attachment, to limit the relative displacement and/or control the position and/or control the movement and/or control the orientation and/or control the magnetic interaction force between the magnetic elements.

Preferably, the first and/or second magnetic elements are affixed to or contained in a supporting element, in particular a textile supporting element also comprising the fastening means. Using such a supporting element, which may be flexible and carries the first or second magnetic element, respectively, facilitates handling of the magnetic elements which may be affixed to or contained in the supporting element in different ways. For example, the first and/or second magnetic element may be affixed to the supporting element using glue and/or may be contained a receptacle, for example a pocket and/or a holder, formed in the supporting element. The magnetic elements may, in particular, even be exchangeable, in particular when using a pocket and/or a holder. The may be loosely or firmly secured in the receptacle Fastening means may be part of the supporting element, but can also still be provided on the magnetic element itself.

Preferably, the tension measurement device comprises multiple first and/or second magnetic elements having different properties influencing the magnetic interaction force such that a combination of at least one first and at least one second magnetic element corresponding to a desired magnetic interaction force strength is selectable, and/or that the tension measurement device comprises multiple constraining elements, in particular multiple spacers, having different properties influencing the magnetic interaction force strength when used with a combination of at least one first and at least one second magnetic element. In concrete embodiments, at least one of the supporting elements may have multiple magnetic elements and/or multiple first and/or second magnetic elements may be provided on multiple supporting elements and/or for a receptacle of the supporting element, the multiple magnetic elements having different properties influencing the magnetic interaction force such that a combination of a first and a second magnetic element corresponding to a desired magnetic interaction force strength is selectable. Therefore, multiple possible first and/or second magnetic elements having different properties are available to select at least one pair of them providing a desired magnetic interaction force and thus a desired indication behavior. Additionally or alternatively, multiple constraining elements constraining the displacement regarding the same aspect (e.g. spacers for minimal distance) can be provided. The tension measurement device can be configured to indicate certain defined tension forces, in particular regarding a desired compression profile for the compression device and/or using indicia. In particular, when at least one of the supporting elements (and thus its magnetic elements) is releasably attachable to the textile compression material or a base material, a suitable supporting element and thus first or second magnetic element can be chosen. If multiple magnetic elements are provided on one supporting element, for example, an orientation of the supporting element may be suitably chosen to enable interaction of a selected magnetic element with a corresponding magnetic element of the selected pair.

In a concrete embodiment, a quadratic supporting element may be provided having four magnetic elements, each on one side of the supporting element. The supporting element which may, for example, be considered a male element, may be rotated such that when mating with a female element, that is, another supporting element, a selected pair of first and second magnetic elements interact to generate the magnetic interaction force.

The different properties may comprise a material composition of the magnetic elements and/or material quantities of the magnetic elements and/or at least one dimension and/or geometric property of the magnetic elements, in particular a dimension and/or geometric property defining a contact area to a respective first and/or second magnetic element, and/or at least one dimension and/or geometric property and/or magnetic property of the constraining elements, and/or an axis of polarity of the magnetic elements and/or, in the case of magnetizable magnetic elements, different magnetizing parameters, in particular electrical current and/or electrical charge, and/or finishes. Please note that, in addition or as an alternative to magnetic design properties of the magnetic elements, the magnetic interaction force may also be adjusted by the relative positioning of the first and second magnetic elements, in particular in the base position. For example, the tension measurement device may comprise multiple spacers as constraining elements, defining different distances between the first and second magnetic elements in the base position, resulting in different magnetic interaction forces depending on which spacer is used. Of course, other constraining elements influencing in particular the base position and/or magnetic fields and thus magnetic interaction force may be used.

In the example laid out above, wherein a graduated supporting element is used, for example, magnetic elements having different lengths with regard to the length of a second magnetic element of a pair to be formed may be used. For example, the magnetic elements may have 25%, 50%, 75% and 100% of the length of the second magnetic element. Depending on the magnetic element chosen to be the first magnetic element, the magnetic interaction force is 100%, 75%, 50% or 25% of the maximum achievable magnetic interaction force, depending on which side of the supporting element is used as male connector to pair with female connector having the second magnetic element. Thus, change in dimension can be used to change the connective strength or in general the strength of the magnetic interaction. However, it should be understood that similar effects can be achieved by changing any combination of the magnetic design properties laid out above, such as material composition, material quantities, mass, size, dimension, geometry, axis of polarity, contact area, spatial positioning, or distance between magnetic elements, for example using spacers such as separators, dividers and the like.

Preferably, indicia are associated with each magnetic element of the magnetic elements having different properties and/or each constraining element, the indicia describing a magnetic interaction force to be overcome when using the respective magnetic element or constraining element. In embodiments, the supporting element may comprise the indicia associated with each magnetic element affixed to it, the indicia describing a magnetic interaction force to be overcome when using the respective magnetic element. Preferably, referring to a situation in which only the magnetic interaction force has to be overcome (magnetic elements attached to unstretched textile compression material) and directly or indirectly contacting first and second magnetic elements are separated to indicate tension, the indicia may indicate the compression resulting from stretching the material by exertion of the defined tension force leading to separation of the first and second magnetic elements. Each magnetic element may be labeled to communicate, for example, the compression range associated with the tension force applied to separate the first and second magnetic element. Thus, selecting suitable first and/or second magnetic elements is facilitated. Aside from the supporting element, indicia may also be provided on other components of the tension measurement device and/or the compression device, for example on the textile compression material and/or magnetic elements themselves.

Indicia may, according to the invention, also be provided for other purposes. For example, at least one indicia element may be provided on a tension measurement device component and/or material, the indicia element being associated with identification and/or selection and/or positioning and/or placement and/or assembly and/or configuration and/or magnetic interaction force of at least one of the magnetic elements and/or constraining elements and/or with an instruction to a user.

The result of providing different magnetic elements selectable as first and/or second magnetic elements to form a pair and/or different constraining elements is an adjustable tensiometer (or modular tension measurement device) as tension measuring device. Magnetic elements could be removed or replaced to change the magnetic interaction force or prevent a user from using other force settings. While change in dimension has been shown as an example for changing the magnetic interaction force, any combination of the magnetic design properties may be changed to result in suitable magnetic interaction forces. The selection of which magnetic design properties to vary may be based on performance, but also design efficiency for tooling and production of the tension measurement device.

As already indicated, preferably, both the first and the second magnetic elements may be affixed to or contained in supporting elements, wherein one supporting element comprises a male connector having the first or second magnetic element and the other supporting element has a female connector having the second or first magnetic element and mating with the male connector to establish the base position. The part of the supporting element where the magnetic element is affixed may thus be designed to be a male or female connector with the respective female or male connector of another supporting element to pair first and second magnetic elements in the base position. In this manner, accurate positioning of the (selected) first and second magnetic elements can be achieved. The connectors are another example of constraining elements.

In this context, it is also preferred that at least one of the connectors comprises an indication portion covered by at least the magnetic element of the other connector such that an indication of the indication portion becomes visible if the first and second magnetic elements are relatively displaced, in particular when the first and second magnetic elements disconnect to be displaced to the second position to indicate the exertion of a defined tension force. For example, such an indication may be a checkmark. The female or male connector may include an area, namely the indication portion, that is hidden from view until relative displacement of the first and second magnetic elements takes place, in particular until they are separated by exerting the defined tension force. When separated, the indication in the indication portion becomes exposed to additionally communicate that the correct tension force has been achieved. If magnetic elements of different sizes are used, the corresponding connectors may also include additional non- magnetic material so that the coverage of the indication portion is identical regardless of which magnetic element is chosen as part of the pair. Thus, the indication portions are indication means, as laid out above.

In an especially preferred embodiment, the tension measurement device may comprise multiple pairs of first and second magnetic elements each characterized by a different magnetic interaction force. Preferably, these pairs are relatively displaced successively when a rising tension force is applied, each covering a certain range of tension force strength and thus applied compression levels. In a concrete embodiment, each pair of first and second magnetic elements is arranged to define a base position and a second position corresponding to a defined tension force such that the pairs of elements successively indicate the respective defined tension forces with rising strength of the tension force applied. In particular, the pairs may be separating successively. This results in a multi-stage tensiometer, where the magnetic interaction force between successive and preferably adjacent pairs of first and second magnetic elements differs, in particular rising monotonically along adjacent pairs. As a tension force is applied by a user, these pairs may successively separate to indicate corresponding defined tension forces. In other words, the pairs can be understood as connected in series. Such a multi-stage tension measurement device has the advantage that upper and lower limits of defined tension forces define certain compression intervals that can specifically be chosen.

At least two adjacent pairs may use the same magnetic element as first and second magnetic element, respectively. That is, if three magnetic elements are considered, the central magnetic element may serve as a second magnetic element in a pair with the rightmost magnetic element adjacent and as a first magnetic element in a pair with the leftmost magnetic element. In this manner, less magnetic elements are required.

In an especially preferred embodiment, each pair of first and second magnetic elements has a display element indicating the defined tension force, in particular as a compression relating to a non-stretched state in the base position, which is revealed when the magnetic elements are relatively displaced into the second position. Preferably, in the case of directly contacting magnetic elements in the base position, the display element, which is an indication means, may be attached to both the first and the second magnetic element of the pair, respectively, wherein the display element is in a folded-up position opposite a viewing direction of a user when the first and second magnetic elements are in the base position such that, when the first and second magnetic elements are separated due to the defined tension force being exerted, the display element is unfolded and visible between the first and second magnetic elements. Such a display element may also concretely define the second position by fully unfolding it, acting as a constraining element. For example, when a first pair of first and second magnetic elements is separated, a first compression range may be revealed which is associated with the defined tension force required for separating the magnetic elements of this pair. Further increasing the tension force separates the next pair, resulting in a display of an increased compression range and thus indicating that the next compression range has been reached. This process continues until the last pair of first and second magnetic elements has been separated such that the highest and last compression level is displayed.

Generally speaking, embodiments of the current invention may also provide alternatives to the above-described hidden visual indicators (indication portion/display element as indication means). Preferably, an indication means indicating the displacement of at least one pair of magnetic elements from the base position and/or plurality of base positions to at least one second position and/or a plurality of second positions may provide at least one audible and/or visual and/or electronic and/or sensory indication that an applied tension force and/or tension forces have been achieved. The indication means thus allows an indication to be triggered upon displacement from the base position to a certain or at least one second position. The indication may be outputted away from the pair itself, in particular when using an electronic indication. For example, the indication means may communicate, in particular wirelessly, with an indication output device. The indication output device may be a smartphone, in which the indication may be generated by a software application (app).

As an alternative to contacting first and second magnetic elements which are separated when a defined tension force is achieved, in this manner indicating this defined tension force, or additionally for another pair of first and second magnetic elements, for at least one pair of first and second magnetic elements the first and second magnetic element may be continuously movable relative to each other against the magnetic interaction force. In this manner, a refined measurement and indication of tension forces can be achieved.

In these embodiments, however, it can be expedient to limit the relative displacement of the first and second magnetic elements, in particular defining a base position and/or preventing their complete separation and/or indicating a certain defined tension force as being reached. Therefore, in preferred embodiments, at least one displacement constraining device, in particular a pin or hook engaging a groove of one of the magnetic elements, may be provided to limit the relative displacement of the magnetic elements to the second position, in particular in the position corresponding to a defined tension force, such that latching of the displacement constraining device, which may be understood as a constraining element, indicates the defined tension force. The displacement constraining device may be a latching device. If relatively free relative displacement of the second magnetic element is to be allowed, a groove, in particular in one of the magnetic elements may, in a concrete embodiment, be tilted and/or funnel-like such that a pin or hook, which is preferably provided on another of the magnetic elements which is displaced relatively, slides guidedly into the groove until an end stopper is contacted. In this manner, latching can be achieved. Preferably, however, the pin or hook is guided in a groove having two end stops, one corresponding to the base position and one corresponding into the second position associated with the defined tension force. In this manner, guided linear displacement is achieved.

It is noted that while the guiding element is generally provided to guide the relative displacement of the first and second magnetic elements, such the guiding element (as part or embodiment of the displacement constraining device) is particularly advantageous in this case since it guides the components of the displacement constraining device towards latching at the end stops of the groove. The displacement constraining device may thus comprise latching means and guiding elements at the same time, since, for example, a pin or hook can be guided in a groove where the respective ends of the groove provide end stoppers defining the base position and the second position associated with the defined tension force, respectively.

The displacement constraining device may preferably also prevent the first and second magnetic elements from fully detaching such that they always stay coupled in use and preferably return to the base position once the applied tension force is no longer present.

In an advantageous concrete embodiment, a first magnetic element is mounted slidably along at least one second magnetic element in a direction essentially parallel to the direction of the tension force such that the magnetic interaction force is a shearing force. In particular, the first magnetic element may be guided between an upper and a lower second magnetic element. In such an embodiment, the tension force applied by a user is working against a shearing force originating in a two or preferably three-layer structure of magnetic materials. A top and bottom magnetic layer, that is, the second magnetic elements, provide a shearing counterforce that drives the first magnetic element back inward if the tension force is removed. Thus, the tensiometer is automatically reset.

In such a configuration, the tension measurement device may also be provided as an adjustable tensiometer where first and/or second magnetic elements may be replaced, in particular by using different pairs of first and second magnetic elements to achieve different defined tension forces at the second position, in particular an end stop of the displacement constraining device. For example, in the three-layer structure described above, the strength of the upper and lower second magnetic elements could be altered by changing the magnetic design properties of the second magnetic elements of the tension measurement device, while the first magnetic element remains the same. For an adjustable compression device various sets of second magnetic elements could be provided to achieve different compression ranges or compression profiles along the limb/extremity. A common example is a gradient compression profile where a greater pressure is desired distally than proximally on the body. Knowing the user's circumference at various points of the limb where bands/straps align, different second magnetic elements could be selected to enable the user to create the desired compression profile, wherein each set of second magnetic elements is assigned to a band or strap. The band or strap, on the other hand, may have a defined first magnetic element attached to it.

It is additionally noted that, in all embodiments allowing continuous movement, indicia may be placed along the path of continuous relative displacement indicating the tension force applied.

In preferred embodiments, at least one of the magnetic elements may be integrated into the textile compression material, in particular as magnetic threads and/or magnetic rods inserted along at least one stitch course and/or stitch wale. This configuration, in which the textile compression material is preferably a knitted fabric or a woven fabric, is particularly advantageous in the case of compression stockings or compression socks as compression devices. Respective pairs of first and second elements, which may also be distanced by a few stitch courses and/or stitch wales, attach together in the textile compression material and separate once a defined tension force is exerted, indicating sufficient compression being exerted. If parts of the textile compression material unfold during this process, indications provided on display areas between the first and second magnetic elements may be revealed such that, additional to a haptic communication, a visual communication to the user is enabled. For example, a pattern may be completed by unfolding textile compression material that was folded up. Depending on their provision along stitch courses and/or stitch wales, longitudinal and crosswise stretching may be measured.

It is noted that the concept described above regarding using multiple pairs of first and second magnetic elements having different associated defined tension forces for their separation may advantageously also be applied to this integrated embodiment, in particular such that indications describing different compression ranges associated with the tension forces are successively revealed on unfolded textile compression material as first and second magnetic elements separate.

In particular regarding the provision of multiple magnetic elements and/or constraining elements from which suitable combinations are to be chosen, the tension measurement device may preferably comprise at least one selection guide element, such as text, symbols, indicia, color coding, geometry, charts, a sensory element, and/or software output, to aid in the tension measurement device use, configuration, orientation, positioning, assembly, constraining element selection, magnetic element selection, magnetic element property selection, and/or material selection to measure at least one desired tension force and/or range of tension forces. The selection guide element may include instruction or coding to help the user select the appropriate elements, pairings, device orientation/positioning, etc. to achieve the desired tension measurement outcome. For example, a chart in the instructions for use (e.g. circumference measurements vs. pressure selection) or other correlations, as also laid out above, may be provided. Another example, as already hinted at above, are indicia elements which may also be provided on the compression device so that when the user applies the garment, the band ends land on the indicia indicating the pairing to use to continue to then apply tension. In essence the compression device may serve as a measuring tape and gives an indication of which pairing(s) to use to achieve different compressions. As a final example, a cell phone software application (app) could be provided as selection guide element to determine the best configuration for the patient described by measured, scanned or entered patient data.

In summary, these advantageous embodiments may be shortly described by the following aspects:
Aspect 1: a tension measurement device for measuring a tension of a textile compression material of a compression device for a limb of a patient, wherein the tension measurement device comprises at least one first and at least one second magnetic element, of which at least one is magnetizable or permanently magnetic and wherein at least one of the magnetic elements is attached or attachable to the textile compression material, wherein the first and the second magnetic elements are relatively displaceable such that tension is measurable depending on a relative displacement of the first and second magnetic elements based on a tension force overcoming a magnetic interaction force between the first and second magnetic elements.
Aspect 2: a tension measurement device according to aspect 1, wherein the first and second magnetic elements are held in a base position corresponding to a base tension state, in particular a non-stretched state, of the textile compression material by the magnetic interaction force such that stretching of the textile compression material resulting in a tension force overcoming at least a defined portion of the magnetic interaction force changes the relative position from the base position to at least one second position to indicate the tension of the textile compression material.
Aspect 3: a tension measurement device according to aspect 2, wherein the magnetic elements are releasably attachable to the textile compression material using a fastening means.
Aspect 4: a tension measurement device according to aspect 3, wherein the fastening means comprise a hook-and-loop fastener, in particular a hook material engaging the textile compression material and/or loops on the textile compression material.
Aspect 5: a tension measurement device according to any of the aspects 2 to 4, wherein the first and second magnetic elements contact directly, or indirectly via a spacer, in the base position such that the exertion of a defined tension force corresponding to the magnetic interaction force results in indication by separation of the contacting magnetic elements.
Aspect 6: a tension measurement device according to one of the aspects 2 to 5, wherein the first and/or second magnetic elements are affixed to or contained in a supporting element, in particular a textile supporting element also comprising the fastening means.
Aspect 7: a tension measurement device according to any of the aspects 2 to 6, wherein it comprises multiple first and/or second magnetic elements having different properties influencing the magnetic interaction force such that a combination of at least one first and at least one second magnetic element corresponding to a desired magnetic interaction force strength is selectable, and/or that it comprises multiple constraining elements, in particular multiple spacers, having different properties influencing the magnetic interaction force strength when used with a combination of at least one first and at least one second magnetic element.
Aspect 8: a tension measurement device according to aspect 7, wherein the different properties comprise a material composition of the magnetic elements and/or material quantities of the magnetic elements and/or at least one dimension and/or geometric property of the magnetic elements, in particular a dimension and/or geometric property defining a contact area to a respective first and/or second magnetic element, and/or at least one dimension and/or geometric property and/or magnetic property of the constraining elements and/or an axis of polarity of the magnetic elements, and/or, in the case of magnetizable magnetic elements , different magnetizing parameters, in particular electrical current and/or electrical charge, and/or finishes.
Aspect 9: a tension measurement device according to aspect 7 or 8, wherein indicia are associated with each magnetic element of the magnetic elements having different properties and/or each constraining element, the indicia describing a magnetic interaction force to be overcome when using the respective magnetic element or constraining element.
Aspect 10: a tension measurement device according to one of the aspects 6 to 9, wherein both the first and the second magnetic elements are affixed to supporting elements, wherein one supporting element comprises a male connector having the first or second magnetic element and the other support element has a female connector having the second or first magnetic element and mating with the male connector to establish the base position.
Aspect 11: a tension measurement device according to aspect 10, wherein at least one of the connectors comprises an indication portion covered by the magnetic element of the other connector such that an indication of the indication portion becomes visible if the first and second magnetic elements are relatively displaced, in particular when the first and second magnetic elements disconnect to be displaced to a second position to indicate the exertion of a defined tension force.
Aspect 12:a tension measurement device according to one of the aspects 2 to 11, wherein it comprises multiple pairs of first and second magnetic elements each characterized by a different magnetic interaction force.
Aspect 13: a tension measurement device according to aspect 12, wherein each pair of first and second magnetic elements is arranged to define a base position and a second position corresponding to a defined tension force such that the pairs of magnetic elements successively indicate the respective defined tension forces with rising strength of the tension force applied.
Aspect 14: a tension measurement device according to aspect 13, wherein at least two adjacent pairs use the same magnetic element as first and second magnetic element, respectively, and/or each pair of first and second magnetic elements has a display element indicating the defined tension force which is revealed when the magnetic elements are relatively displaced into the second position.
Aspect 15: a tension measurement device according to one of the aspects 2 to 14, wherein it comprises an indication means indicating the displacement of at least one pair of magnetic elements from the base position and/or plurality of base positions to at least one second position and/or a plurality of second positions, which provides at least one audible and/or visual and/or electronic and/or sensory indication that an applied tension force and/or tension forces have been achieved.
Aspect 16: a tension measurement device according to one of the preceding aspects, wherein, for at least one pair of first and second magnetic elements, the first and second magnetic elements are continuously moveable relative to each other against the magnetic interaction force.
Aspect 17: a tension measurement device according to aspect 16, wherein at least one displacement constraining device, in particular a pin or hook engaging a groove of one of the magnetic elements, is provided to limit the relative displacement of the magnetic elements to the second position, in particular in a position corresponding to a defined tension force, such that latching of the displacement constraining device indicates the defined tension force.
Aspect 18: a tension measurement device according to aspect 16 or 17, wherein a first magnetic element is mounted slidably along at least one second magnetic element in a direction essentially parallel to the direction of the tension force such that the magnetic interaction force is a shearing force.
Aspect 19: a tension measurement device according to one of the preceding claims, wherein at least one of the magnetic elements is integrated into the textile compression material, in particular as magnetic threads and/or magnetic rods inserted along at least one stitch course and/or stitch wale.

The basic concepts of the current invention may also be applied to other devices, in particular closure systems or even the creation of self-adjusting tensiometers. For example, a self-adjusting attachment device for a band of a compression device having a textile compression material is conceivable. Such a self-adjusting attachment device would comprise:
- a first magnetic element affixed or affixable to a band end of the band that is to be attached, and
- a wedge-shaped second magnetic element affixed or affixable to a portion of the compression device where the band is to be attached, the second magnetic element having a sliding surface inclined with respect to an opposite, affixing surface affixed or affixable to the portion, such that the first magnetic element positioned slidably on the sliding surface, due to a magnetic interaction force between the first and second magnetic elements, experiences a lesser friction force when positioned on the thin far end of the second magnetic element than when positioned closer to the near, thick end of the second magnetic portion.

Preferably, the first magnetic element may also be wedge-shaped and/or be shorter than the sliding surface of the second magnetic element.

To attach the band, the first magnetic element is placed on the far, thin end of the sliding surface. The resulting tension in the compression device due to stretching to the far end pulls the first magnetic element attracted by the second magnetic element up the slope of the sliding surface. The first magnetic element is displaced along the sliding surface until equilibrium is reached, that is, the friction force influenced by the magnetic interaction force corresponds to the compression force of the compression device.

Please note that this self-adjusting attachment device also functions as a tension measurement device since tension force applied to the band results in readjustment and change of the relative position of the first magnetic element on the second magnetic element. That is, the position of the first magnetic element on the second magnetic element indicates an applied tension force.

The component of the self-adjusting attachment device having the second magnetic element may also be termed a magnetic landing. The first and/or the second magnetic element may be exchangeable such that, for example, through selection of band and/or magnetic landing magnetic materials of different magnetic design properties, the equilibrium and resulting compression can be modified without altering the application process. Both the landing and band end magnetic elements can be positioned on compression devices where needed or using indicia on the compression device.

Further advantages and details of the current invention become apparent from the following description of preferred embodiments, taken in conjunction with the drawings, in which
- Fig. 1: illustrates the magnetic interaction between two magnetic elements,
- Fig. 2: is a graph showing the magnetic interaction force dependence on the distance between the magnetic elements,
- Fig. 3: is a top view of a first embodiment of a tension measurement device according to the invention,
- Fig. 4: is a cross-sectional side view of a first component of the tension measurement device of Fig. 3,
- Fig. 5: is a cross-sectional side view of a second component of the tension measurement device according to Fig. 4,
- Fig. 6: shows a second embodiment of a tension measurement device according to the invention with no applied tension force,
- Fig. 7: is a side view corresponding to Fig. 6,
- Fig. 8: shows the second embodiment in a state where a first tension force is applied,
- Fig. 9: is a side view corresponding to Fig. 8
- Fig. 10: shows the second embodiment in a state where a second tension force is applied,
- Fig. 11: is a side view corresponding to Fig. 10
- Fig. 12: shows the second embodiment in a state where a third tension force is applied,
- Fig. 13: is a side view corresponding to Fig. 12
- Fig. 14: shows components of a third embodiment of a tension measuring device according to the invention,
- Fig. 15: shows cross-sectional side views of the components of Fig. 10,
- Fig. 16: shows the third embodiment in a base position with no applied tension force,
- Fig. 17: shows the third embodiment in a cross-sectional side view when a defined tension force is applied,
- Fig. 18: illustrates a possible integration of magnetic elements into a stitched or woven fabric,
- Fig. 19: shows a first embodiment of a compression device according to the invention,
- Fig. 20: shows components of an embodiment of a self-adjusting attachment device,
- Fig. 21: shows the components of Fig. 16 in a side view,
- Fig. 22: shows a first state of the self-adjusting attachment device,
- Fig. 23: shows a second state of the self-adjusting attachment device,
- Fig. 24: shows a fourth embodiment of a tension measurement device according to the invention,
- Fig. 25: is a side view of the fourth embodiment in a base position with no applied tension force,
- Fig. 26: is a side view of the fourth embodiment in a second position with applied tension force,
- Fig. 27: shows a second embodiment of a compression device according to the invention,
- Fig. 28: illustrates a selection guide element of the second embodiment,
- Fig. 29: shows a compression material prepared for a force and/or elongation measurement,
- Fig, 30: shows the application of a fifth embodiment of a measurement device according to the invention to the compression material of Fig. 29,
- Fig. 31: is a top view onto the fifth embodiment in a first state,
- Fig. 32: is a top view onto the fifth embodiment in a second state,
- Fig. 33: is a top view onto the fifth embodiment in a third state,
- Fig. 34: illustrates a modification of the fifth embodiment in a first state,
- Fig. 35: shows the modified the fifth embodiment in a second state, and
- Fig. 36: shows a scale used in the modified fifth embodiment.

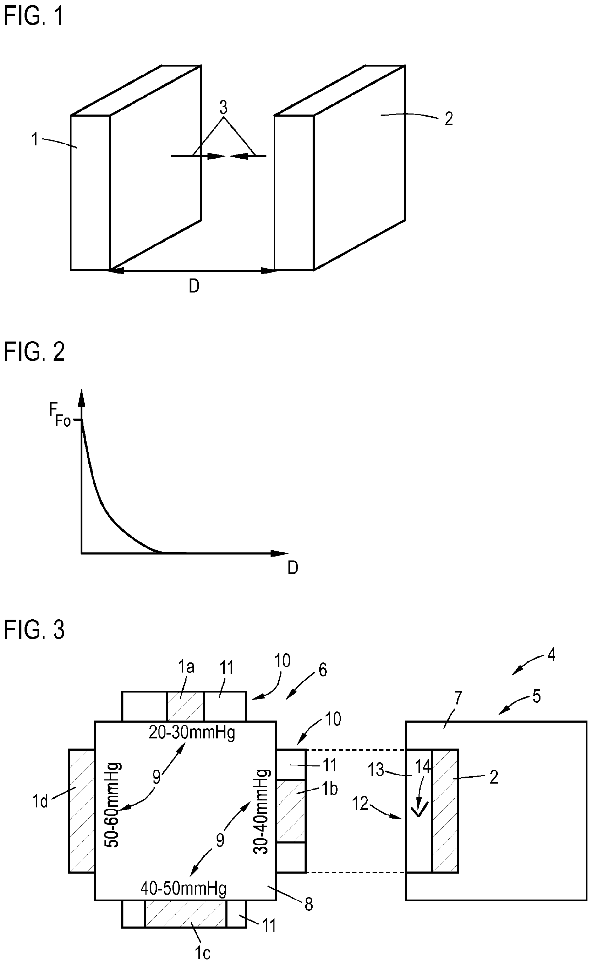

Fig. 1 shows a perspective view of a first magnetic element 1 and a second magnetic element 2, in this case both permanent magnets (an alternative would be temporally magnetizable magnetic elements, in particular by using an electric current). The magnetic elements 1, 2 are spaced by a distance D and experience, as known in the art, an attractive interaction force indicated by arrows 3 if their polarities are opposite. The magnetic interaction force which has to be overcome to separate the magnetic elements 1 and 2 decreases as the paired magnetic elements 1 and 2 become further apart, as shown in the graph of Fig. 2. The closer the magnetic elements 1, 2 are returned together, the more likely they are to reconnect, that is, directly contact. To separate two directly contacting magnetic elements 1, 2, a force F0 needs to be exerted.

The magnetic interaction force described by arrows 3 is influenced by certain properties of the magnetic elements 1, 2 which may be called magnetic design properties (MDP). The strength of the magnetic interaction force is further influenced by the relative displacement of the first and second magnetic element 1, 2. For example, if the contact area between the first and second magnetic elements 1, 2 is reduced by 50% by changing the height of one of the first and second magnetic elements 1, 2 by 50%, the resulting magnetic interaction force to separate the magnetic elements 1, 2 is thus reduced by approximately 50%. This example shows how changes in dimensions of the magnetic elements 1, 2 can be used to change the connective strength. However, similar effects can also be achieved by changing other magnetic design properties, in particular additionally, such as material composition, material quantities, mass, size, dimension, geometry, axis of polarity, contact area and the like. The relative placement of the first and second magnetic elements 1, 2 also influences the magnetic interaction force as illustrated by Fig. 2. If a lower magnetic interaction force is desired, for example, spacers such as separators or dividers or other constraining elements may be used to provide indirectly contacting first and second magnetic elements 1, 2.

When donning a compression device, for example a compression garment, the user exerts a tension force acting on the textile compression material of the compression device to stretch it and uses a closure system to keep the textile compression material in this stretched state such that it applies compression according to the tension force to the limb. In the following embodiments, tension measurement devices are proposed in which this user-initiated tension force acts against magnetic interaction forces of a pair of first and second magnetic elements 1, 2, wherein the magnetic elements 1, 2 are positioned such that they can be displaced relatively to each other when a tension force is exerted. The resulting displacement is used to indicate the tension force (and thus in particular an associated compression level).

Fig. 3 - 5 illustrate a first embodiment of a tension measurement device 4 according to the invention. As can be seen, the tension measurement device 4 comprises two components 5, 6 each having a supporting element 7, 8 on which magnetic elements 1a, 1b, 1c, 1d and 2 are affixed. Only one magnetic element 2, acting as a second magnetic element, is provided on supporting element 7, while four magnetic elements 1a, 1b, 1c and 1d are provided on supporting element 8. Depending on which magnetic element 1a, 1b, 1c, 1d is chosen as first magnetic element 1 to pair with the second magnetic element 2, different magnetic interaction forces arise since the magnetic elements 1a, 1b, 1c and 1d differ in their length, providing different contact areas. In this embodiment, if the magnetic element 2 has a length of 100%, the magnetic element 1c has 75%, the magnetic element 1b has 50% and the magnetic element 1a has 25%. The supporting element 8 comprises indicia 9 associated with each of the magnetic elements 1a, 1b, 1c and 1d, in this case indicating a compression range associated with a defined tension force needed to overcome the directly contacting respective first magnetic element 1a, 1b, 1c and 1d from the second magnetic element 2.

As can further be seen from Fig. 3, the component 6 has male connectors 10 each comprising one of the magnetic elements 1a, 1b, 1c and 1d. In the connectors 10 of the magnetic elements 1a, 1b and 1c, non-magnetic material 11 has been added so that the total length of the connectors 10 correspond to the total length of the magnetic elements 1d (100%).

The reason is that the corresponding female connector 12 of the component 5 comprises the opening and magnetic element 2 of the same length (100%). As an indication means, an indication portion 13 having an indication 14, in this case a checkmark, thereon. The non-magnetic material 11 of the male connectors 10 ensures that the whole indication portion 13 is covered when the connectors 10, 12 mate, i.e. the selected first magnetic element 1a, 1b, 1c, 1d directly connects with the second magnetic element 2.

If, for example, the magnetic element 1b has been chosen to pair with magnetic element 2, as indicated in Fig. 3, a tension force corresponding to the compression level of 30 - 40 mmHG leads to separation of the first and second magnetic elements 1b, 2, exposing the checkmark and providing additional visual indication that the defined tension force needed for separation has been exerted.

The component 6 may be rotated to select any of the magnetic elements 1a, 1b, 1c and 1d as first magnetic element 1 to pair with magnetic element 2.

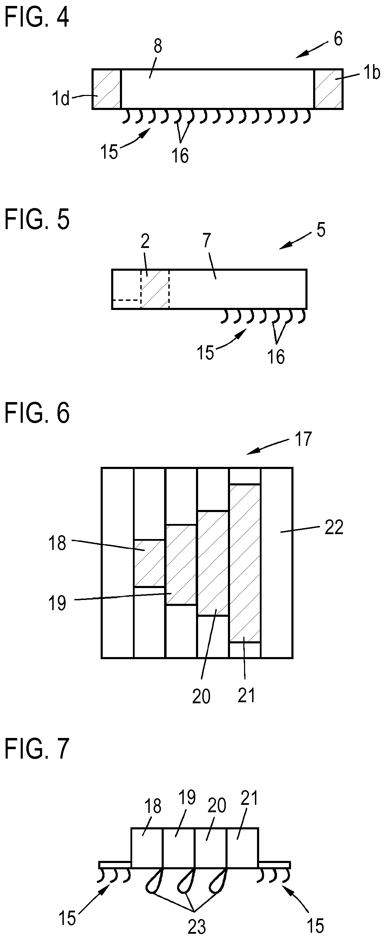

For compression measurement on a compression device, the chosen magnetic element 1a, 1b, 1c or 1d is directly contacted with magnetic element 2 by mating the corresponding connector 10 with the connector 12. The pair of the chosen first magnetic element 1a, 1b, 1b or 1d and the second magnetic element 2 is then in a base position. The tension measurement device 4 is then attached to the textile compression material of the compression device, wherein the textile compression material of the compression device is in an unstretched state. For example, the tension measurement device 4 may be attached to a body portion of a compression garment or, preferably, onto a band of the compression device which is made of the textile compression material. To facilitate attachment of the tension measurement device 4 to the textile compression material, as can be seen in Fig. 4 and Fig. 5, the supporting elements 7, 8 comprise fastening means 15 on their attachment sides, in this case hook material 16 to engage with the textile compression material directly or with a corresponding loop material on the textile compression material. Thus, the fastening means 15 may be hook-end-loop fasteners. As can be seen from Fig. 5, specifically, an area where the first magnetic element 1a, 1b, 1c or 1d contacts the second magnetic element 2 is kept free of hook material 16 to allow the textile compression material below the contact area to expand/stretch so that displacement of the first and second magnetic elements 1a, 1b, 1c, or 1d, and 2, in this case separation, is enabled. In other words, the fastening means 15 are not continuous along the length of the tension measurement device 4 such that a portion of the textile compression material is free to elongate to allow the magnetic materials to separate. While, in the case of a highly elastic textile compression material, the distance between the hook materials 16 may be small, in a very low stretch textile compression material further separation of the hook material 16 may be necessary.

It is noted that supporting elements 7 or 8 having multiple magnetic elements 1, 2 are not the only way to provide an adjustable tension measurement device 4. It is also possible to provide multiple components 5 and/or 6 having magnetic elements 1, 2 of different magnetic design properties, each with like male or female connectors 10, 12. Of course, both approaches may also be combined.

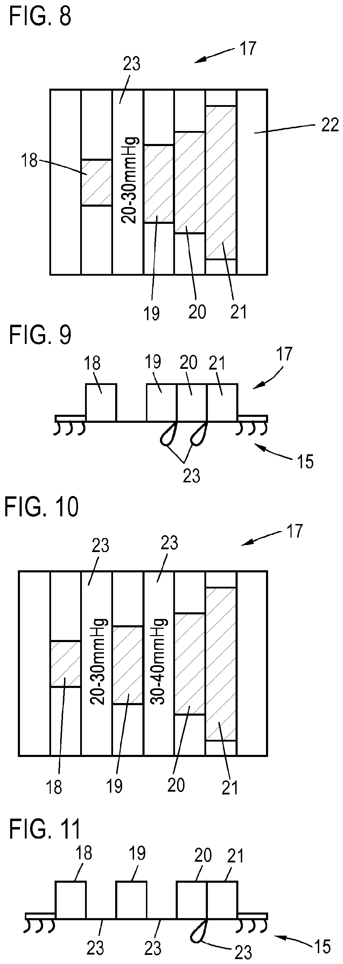

Fig. 6-13 illustrate a second embodiment of a tension measurement device 17 according to the invention. On a flexible base material 22 acting as a supporting element, four magnetic elements 18, 19, 20, 21 are arranged. In their base position shown in Fig. 6, these magnetic elements 18, 19, 20 and 21 all pairwise contact directly. As in all concrete embodiments described here, the magnetic elements 18, 19, 20 and 21 are permanent magnets. The size of the magnetic elements 18, 19, 20, 21 increases along a length of the tension measurement device 17, wherein, corresponding to the first embodiment, if the magnetic element 21 has a length of 100%, the magnetic element 20 has a length of 75%, the magnetic element 19 has a length of 50% and the magnetic element 18 has a length of 25%.

All pairs of directly adjacent magnetic elements, that is, pairs 18, 19; 19, 20; 20, 21 act as pairs of first and second magnetic elements 1, 2. In the first pair, the magnetic element 18 acts as first magnetic element 1 and the magnetic element 19 acts as second magnetic element 2, in the second pair, the magnetic element 19 acts as first magnetic element 1 and the magnetic element 20 acts as second magnetic element 2, and in the third pair, the magnetic element 20 acts as first magnetic element 1 and the magnetic element 21 as second magnetic element 2. As can be seen from the side view of Fig. 7, the respective pairs of magnetic elements 18, 19, 20, 21 are each coupled by a folded-up portion of the base material 22 acting as a display element 23, in this case a combination of an indication means and a constraining element. When magnetic elements 18, 19, 20, 21 are separated, corresponding display elements are revealed between them and define a second, separated position of the respective pair. The display element 23 comprises an indication of a compression range corresponding to the defined tension force to separate the magnetic elements 18, 19, 20, 21 of the respective pair. The tension measurement device 17 can thus be understood as a multi-stage tensiometer where the magnetic interaction force between magnetic pairing 18, 19 is less than between magnetic pairing 19, 20, which again is less than between magnetic pairing 20, 21. As a tension force is applied, the 18, 19 pairing separates first displaying an associated compression range corresponding to the applied tension force. If tension force is further applied, the pairing 19, 20 separates, displaying an increased compression range giving an indication that the next compression range has been achieved. This also serves as an "over-tightened" indicator mechanism. Exertion of even larger tension force finally separates the pairing 20, 21 so that the last and highest compression range is displayed via display element 23.

For the tension measurement device 17, also fastening means 15 can be provided to releasably attach the tension measurement device 17 to a textile compression material of a compression device, in particular a band. However, the tension measurement device 17 can also be permanently attached or integrated into a band of a compression device. For example, tension measurement devices 17 could be sewn into a series of compression bands where they become part of the fabric of the band or compression device. Upon relief of the applied tension force, the separation distance of the magnetic elements 18, 19, 20, 21, due to the limitation provided by the display elements 23, is small enough so that the magnetic interaction force remains sufficient to automatically reconnect the pairings. This, of course, also holds true for a releasably attachable tension measurement device 17.

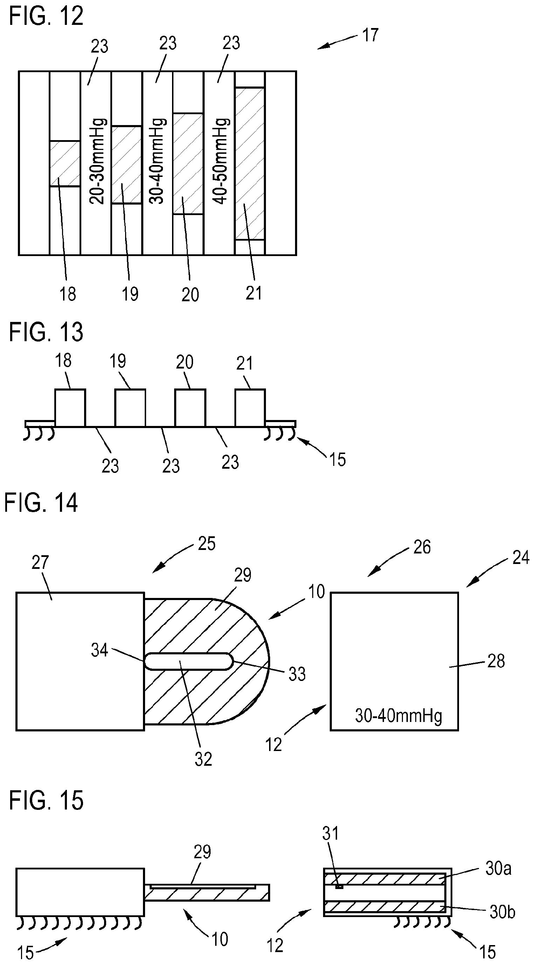

Fig. 14 - 17 illustrate a third embodiment of a tension measurement device 24 according to the invention. The tension measurement device 24 again consists of two components 25, 26, wherein each component 25, 26 may comprise a supporting element 27, 28 to which magnetic elements 29 or 30a, 30b are affixed, respectively. The component 25 has a male connector 10 again pairing with a female connector 12 of the component 26.

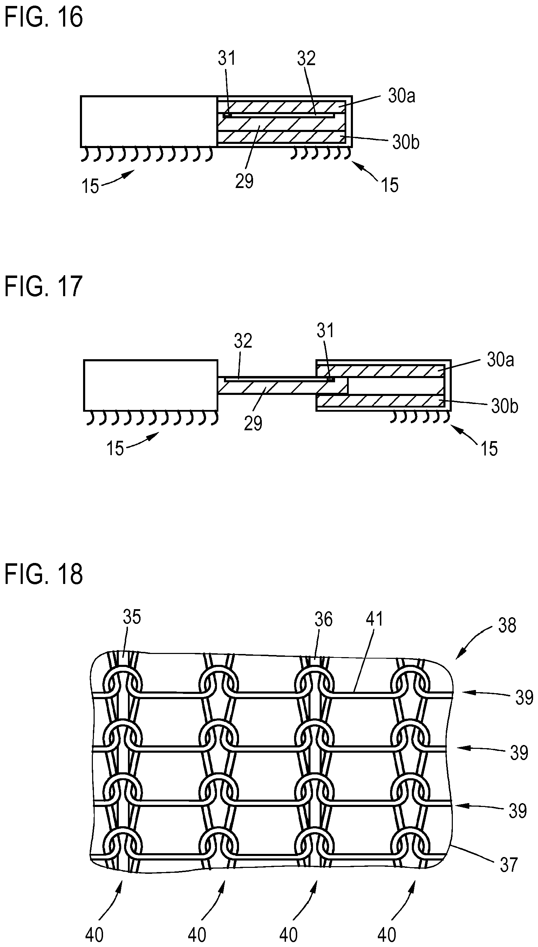

As can be seen from the cross-sectional side view of Fig. 15, while the male connector 10 only comprises the magnetic element 29 (acting as first magnetic element 1), the female connector comprises an upper second magnetic element 30a and a lower second magnetic element 30b between which the first magnetic element 29 is to be slidingly inserted. To limit relative displacement of the magnetic elements 29 and 30a, 30b in a defined manner, the tension measurement device 24 also comprises a displacement constraining device having a pin 31 protruding from the upper second magnetic element 30a and a groove 32 in the center of the magnetic element 29 receiving the pin 31 guidingly as the connectors 10, 12 mate. The ends of the groove 32 provide end stoppers 33, 34 for the pin 31, wherein the end stop 34 defines a base position of the magnetic elements 29, 30a, 30b and the end stop 33 defines a second position corresponding to a defined tension force and prevents detaching of the component 25 from the component 26. Thus, the elements 28, 30, 31, 32, 33 and 34 are all constraining elements. In this third embodiment, the tension force works against the shearing force between the three layers of magnetic materials provided by the magnetic elements 29, 30a and 30b. The top and bottom magnetic elements 30a, 30b of the female connector 12 provide a shearing counter force that draws the male connector 10 back to the base position shown in Fig. 16 where the pin 31 latches with the end stopper 34. This design also leads to automatic reset of the tension measurement device 24 when no tension force is applied. If a tension force is applied, a magnetic element 29 is displaced continuously and linearly from the base position due to the pin 31 guided in the groove 32. When a defined tension force is exerted, the pin 31 latches with the end stopper 33, indicating the defined tension force has been reached.

While the component 25 may be releasably or permanently attached to a band of the compression device, due to the fastening means 15, again hook material 16, preferably the component 26 and thus the second magnetic elements 30a, 30b may be exchangeable. Corresponding defined tension forces may be indicated on the supporting element 28, as shown in Fig. 14.

This means that the strength of the female connector 12 can be altered by changing the material design properties of the second magnetic elements 30a, 30b while the male connector 10 remains the same. For an adjustable compression device, various female components 26 may be provided to achieve different compression ranges or compression profiles along the limb.

It is noted that it is also possible to add indicia indicating the strength of the tension force applied on the surface of the first magnetic element 29, the indicia being revealed when the first magnetic element 29 is pulled out from between the second magnetic elements 30a and 30b and acting as indication means.

As already described, tension measurement devices according to the invention, in particular the second embodiment, may be at least partly integrated into compression devices. Fig. 18 illustrates the integration of magnetic elements 35, 36 into a stitched or woven fabric 37 of a textile compression material 38. For the sake of simplicity, compression threads are not shown.

The fabric 37 comprises stitch courses 39 and stitch wales 40. In the embodiment shown in Fig. 18, rod-like magnetic elements 35, 36 have been inserted into stitch wales. Alternatively, it is also possible to insert magnetic rods along stitch courses or even use a magnetic thread as one of the threads 41.

In the embodiment of Fig. 18, the magnetic element 35 may act as first magnetic element 1 and the magnetic element 36 may act as second magnetic element 2. In this case, at least one stitch wale without a magnetic element 35, 36 is positioned between the magnetic element 35, 36, so that upon connecting the magnetic elements 35, 36 due to the magnetic interaction force, the fabric 37 between these magnetic elements 35, 36 is folded up and stretches out on separation of the magnetic elements 35, 36, in some embodiments displaying a certain pattern or indication of the tension force applied or a corresponding compression level/compression range. In particular, a functionally similar embodiment to the second embodiment may be realized.

It is noted that depending on whether the magnetic elements 35, 36 (or magnetic threads 41) are oriented along the stitch courses 39 or the stitch wales 40, tension applied in longitudinal direction or crosswise direction can be measured.

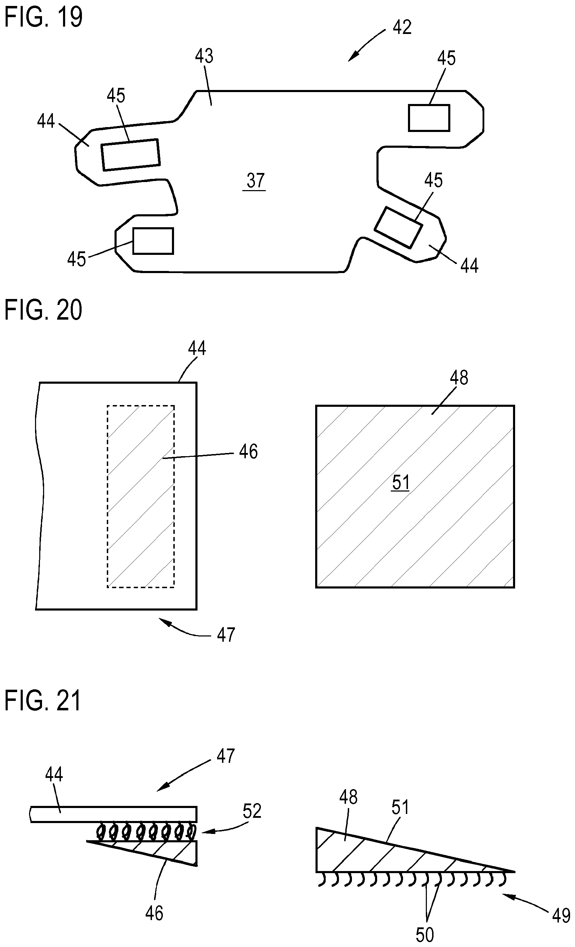

Fig. 19 shows an embodiment of a compression device 42 according to the current invention. In this case, the compression device 42 is a compression wrap, but it may also be a compression garment or another compression therapy device.

The compression device 42 is made of a textile compression material 37 and comprises a body portion 43 from which bands 44 extend. The body portion 43 may wrap at least partly around a limb of a patient and the bands 44 may wrap around the limb and body portion 43 to apply compression to the limb. The bands 44 may attach releasably at different positions of the body portion 43 and/or the bands 44.

In this embodiment, a tension measurement device 45 according to the current invention is associated with each band 44, in particular attached to it or integrated into it. Preferably, the second embodiment (tension measurement device 17) or the third embodiment (tension measurement device 24) may be used.

The tension force applied to different bands 44 when donning the compression device 42 may result in different compression to different areas of the limb. A predetermined compression profile along the extremity, that is, the limb, may be realized using the tension measurement devices 45. To achieve this, tension measurement devices 45 whose first and/or second magnetic elements differ in their magnetic design properties may be used. For example, if a gradient compression profile where greater compression is desired distally than proximally on the body is used as a pre-determined compression profile, the user's circumference where the bands 44 wrap around the limb may be measured.

According to the user circumferences, corresponding tension measurement devices 45 having suitable magnetic interaction forces may be chosen, for example compression measurement devices 45 having different female components 26. Compression profile standards such as RAL could be achieved along with any other compression profile desired. If the compression device 42 is further marked with indicia to measure the user's circumference as the compression device is applied, the user or manufacturer can easily reference a force-circumference-compression matrix to select the appropriate strength of the compression measurement devices 45 for each area covered by the compression device 42. Additionally, preset configuration can be communicated in the instructions for use for standard-size, "off-the-shelf" compression devices to change the compression levels and/or profile.

The tension measurement devices according the current invention also provide a means to notify the user when the tension has dissipated due to garment migration or loss of power in the textile elasticity, since the tension may, in this case, not be upheld or compression is not exerted on the limb. This may result in permanent separation of first and second magnetic elements or reconnection of separated magnetic elements. In the event that the textile "stretches out", the tension measurement device may be removed and re-attached since it is less subject to aging effects than the textile.

Fig. 20 - 23 illustrate a self-adjusting attachment device for a band 44 of a compression device. The compression device, and in particular the band 44, comprises textile compression material. A fist magnetic element 46 is affixed or affixable to a band end 47 of the band 44, wherein, in this embodiment, the first magnetic element is wedge-shaped. A wedge-shaped second magnetic element 48 is affixed to a portion of the compression device where the band end 47 is to be attached, in this embodiment using fastening means 49 in the form of hook material 50. The second magnetic element 48 has a sliding surface 51 opposite an affixing surface where the hook material 50 is provided.

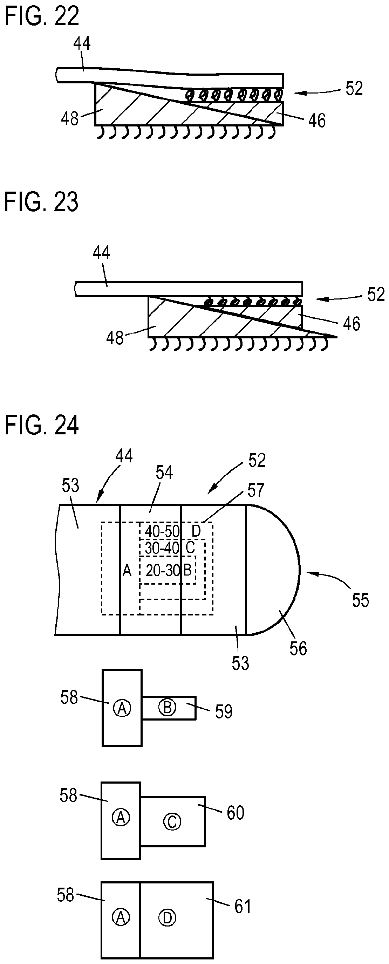

To attach the band 44, the band end 47 with the first magnetic element 46 is positioned at the far end of the sliding surface 51 as shown in Fig. 22. In this area, due to the thinner second magnetic element 48, the magnetic interaction force is lower and thus a resultant friction against sliding is reduced. If, now, the return tension force exceeds the friction force influenced by the magnetic interaction, the first magnetic element 46 will slide along the sliding surface 51 towards the near edge of this so-provided landing where the magnetic interaction force and thus the friction force steadily becomes stronger. The first magnetic element 46 and thus the band 44 slides until equilibrium is achieved.

Band end magnetic elements 46 of different magnetic design properties may be selected to modify the equilibrium and the resulting tension force without altering the application process. Both the second magnetic element 48 as a landing and the band end magnetic element 46 can be positioned on compression devices where needed or via indicia on the compression device.

Figs. 24 - 26 illustrate a fourth embodiment of the tension measurement device 52 according to the invention, which is to be applied to a band 44 of a compression device. As can be seen, the band 44 comprises sections of a magnetic material 53 between which a section of elastic compression material 54 is provided in a longitudinal direction. The band end 55 having hook material 56 is also shown.

Indicia 57 are printed on materials 53, 54 to guide a user in placing magnetic elements 58, 59, 60 and 61 illustrated below the band 44, wherein the first magnetic element 58 itself having indicia labeling it "A", is shown multiple times to illustrate the relative sizes regarding second magnetic elements 59, 60 and 61, having indicia labeling them "B", "C", and "D", respectively. The pair of magnetic elements 58 and 59 has a magnetic interaction force which, when separating the pair of magnetic elements 58, 59, corresponds to an applied tension resulting in a compression range of 20 - 30 mm Hg. For the pairs 58, 60 and 58, 61, the corresponding tensions and compression ranges are 30 - 40 mm Hg and 40 - 50 mm Hg, respectively. As can be seen from Fig. 24, these values are also printed onto the material 54 associated with the label "B", "C" and "D" as part of the indicia 57. If the first magnetic element 58 and a selected one of the second magnetic elements 59, 60 or 61 are placed according to the indicia 57 onto the materials 53, 54, the materials 53 interact with the magnetic elements 58, 59, 60, 61 to provide a magnetic fastener as fastening means.

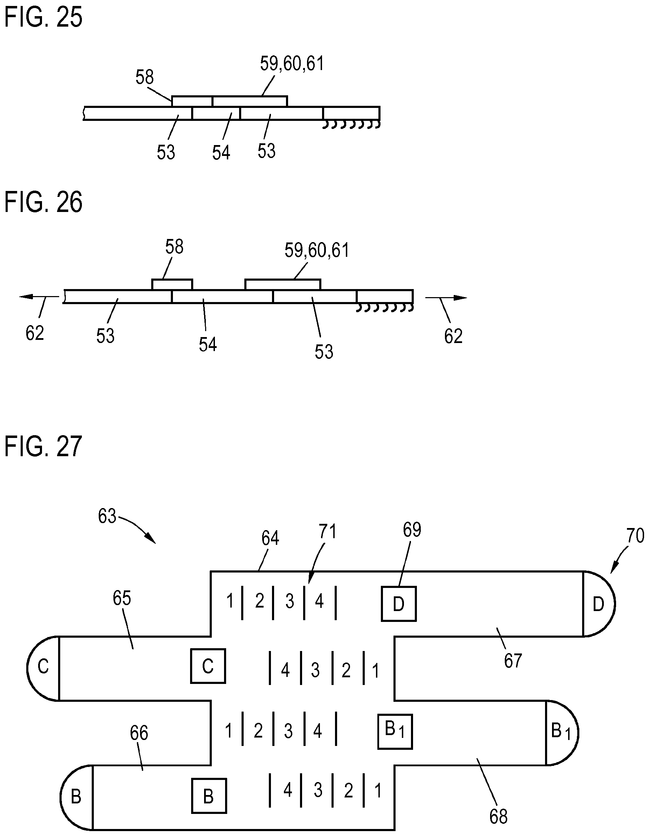

As can be seen from Fig. 25, wherein a pair of a first magnetic element 58 and a second magnetic element 59, 60 or 61 has been attached to the band 44 according to the indicia 57, the contact area between the first magnetic element 58 and the second magnetic element 59, 60, 61 is centered above the compression material 54, which is elastic.

If a tension force is applied to the band 44, as illustrated by the arrows 62 of Fig. 26, the tension force applied counteracts the magnetic interaction force between the pair and, once a tension force corresponding to the magnetic interaction force is reached, the material 54 is stretched and the magnetic elements 58 and 59, 60 or 61 are separated, indicating that the corresponding compression level/range, which is also indicated by the indicia 57, has been reached.

Fig. 27 shows a second embodiment of a compression device 63 according to the invention. As can be seen, the compression device 63 again comprises a body portion 64 and, in this case, four bands 65, 66, 67 and 68 extending from the body portion 64. The compression device comprises multiple indicia 69, 70 and 71.

The indicia 69 mark positions where first and second magnetic elements (not shown) are to be placed once they have been selected as outlined below. The indicia 70 are band/limb region indicators, in this case B, B₁, C and D. The indicia 71 are size indicia indicating a circumference of a limb or body part around which the body portion 64 and the bands 65, 66, 67 and 68 may be wrapped. In other words, the indicia 71 help to determine the circumference of the limb or body part.

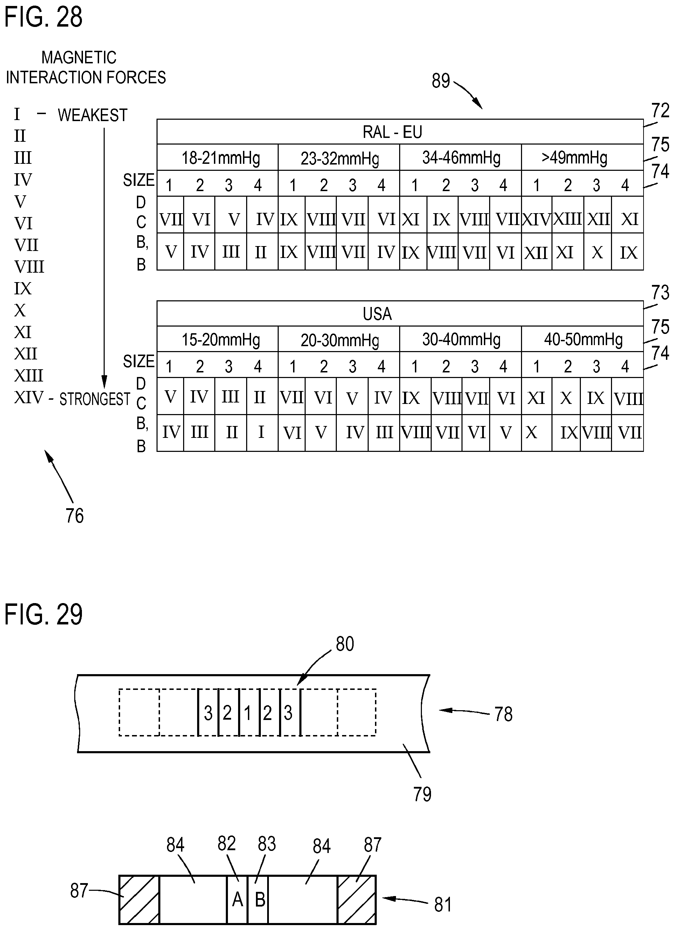

Fig. 28 shows an example of a selection guide element 89, in this case comprising two tables 72, 73, providing correlations between circumferences (size), see row 74, and compression values, see row 75 with magnetic interaction forces and thus pairs of first and second magnetic elements to be chosen. As can be seen from the chart 76 on the left of Fig. 28, the magnetic interaction forces increase from I to XIV. As can further be seen from the tables 72 and 73, the band tensions in different limb regions are different. The table 72 corresponds to the compression profile standard RAL-EU, whereas the table 73 refers to the compression profile standard of the USA.

If a patient is to be treated using a predetermined compression profile, the circumferences of the limb may first be determined using the indicia 71, whereafter these circumferences and the compression values according to the predetermined compression profile may be used to consult one of the tables 72, 73, depending on which standard is used, to select pairs of first and second magnetic elements for each band 65, 66, 67, 68. The selected magnetic elements may then be correctly placed using the indicia 69. Once the pairs of magnetic elements are selected and placed, they can be used to measure if the correct compression level according to the predetermined compression profile is applied to the limb.

Figs. 29 - 33 illustrate a fifth embodiment of a measurement device 77 according to the invention. In this case, force and elongation, that is, displacement, may be measured.

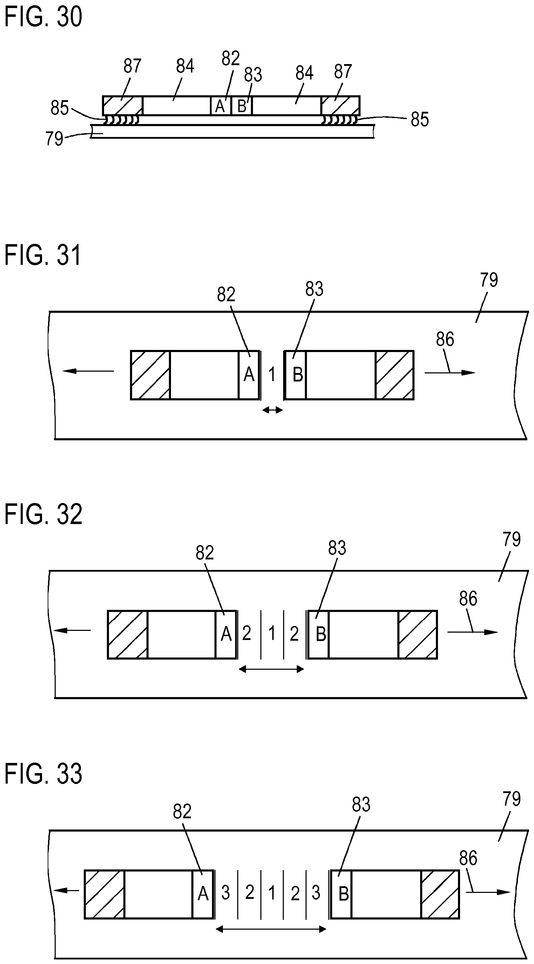

As can be seen from Fig. 29, on a compression material 79, multiple indicia 80 are provided, serving partly to attach a measuring assembly 81 to the material 79, partly to indicate applied tension force/elongation. The measurement assembly 81 comprises a first magnetic element 82 and a second magnetic element 83 which are directly contacting in a base position. The magnetic elements 82, 83 are respectively fixed to a supporting element 84, indirectly attaching them to a fastening means 87 having hook or loop material 85 to attach the measurement assembly 81 to the material 79, as can be seen in Fig. 30. The measurement assembly 81 with the magnetic elements 82, 83 contacting, that is, in a base position, is releasably attached to the compression material 79 in an unstretched state according the corresponding indicia 80.

If, now, a tension force is applied to the compression material 79, as indicated by arrows 86 in Fig. 31 - 33, the magnetic elements 82, 83 are separated once the magnetic interaction force holding them in contact in the base position is surpassed. This situation is shown in Fig. 31, such that now the indicia "1" of the indicia 80 is visible, indicating a still low force strength/elongation. If the tension force according to the arrows 68 is further increased, additional indicia 80, in the case of Fig. 32 and 33 "2" and "3", become visible, indicating this increased tension force and thus elongation.

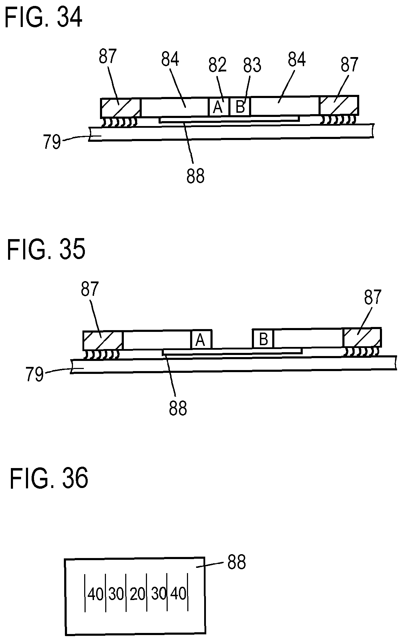

Fig. 34 - 36 illustrate a modification of this fifth embodiment. In this modified fifth embodiment, at least the indicia 80 acting as indication means, that is, indicating the applied force/displacement, on the material 79 may be omitted since a separate scale 88, shown in a top view in Fig. 36, is provided and may be placed centrally below the magnetic elements 82, 83 and the supporting elements 84. In this case, the scale 88 is specific for the underlying limb circumference, compression material 79, and/or the pair of magnetic elements 82, 83 used. It is noted that in the fifth embodiment, the measurement assembly 81 may also comprise spacers as constraining elements to be placed between magnetic elements 82, 83. Scale 88 may be held in place via a magnetic interaction force with elements 82 and 83.

It is noted that, of course, multiple first and/or second magnetic elements 82, 83 may also be provided in the case of the fifth embodiment, allowing different measurement configurations. In the case of the modified fifth embodiment, multiple scales may be provided for the different limb circumferences and/or combinations of elements/materials. In all described embodiments, multiple first and/or second magnetic elements may be provided. According to the invention, all described embodiments comprise a guiding element to guide the relative displacement of the first and second embodiment as a constraining element.

## Claims

1. Measurement device (4, 17, 24, 45, 52, 78) for measuring a tension and/or a displacement of a compression material (37, 54, 79) of a compression device (42, 63) or a compression force applied to a patient and/or a displacement of the compression device (42, 63), **characterized in that** the measurement device (4, 17, 24, 45, 52, 78) comprises at least one first and at least one second magnetic element (1, 1a, 1b, 1c, 1d, 2, 18, 19, 20, 21, 29, 30a, 30b, 35, 36, 58, 59, 60, 61, 82, 83), wherein the first and the second magnetic elements (1, 1a, 1b, 1c, 1d, 2, 18, 19, 20, 21, 29, 30a, 30b, 35, 36, 58, 59, 60, 61, 82, 83) are relatively displaceable to each other such that the tension and/or force is measurable depending on a relative displacement of the first and second magnetic elements (1, 1a, 1b, 1c, 1d, 2, 18, 19, 20, 21, 29, 30a, 30b, 35, 36, 58, 59, 60, 61, 82, 83) to each other based on a tension and/or force overcoming a magnetic interaction force between the first and second magnetic elements (1, 1a, 1b, 1c, 1d, 2, 18, 19, 20, 21, 29, 30a, 30b, 35, 36, 58, 59, 60, 61, 82, 83), and at least one guiding element to guide the relative displacement of the first and second magnetic elements (1, 1a, 1b, 1c, 1d, 2, 18, 19, 20, 21, 29, 30a, 30b, 35, 36, 58, 59, 60, 61, 82, 83).

2. Measurement device (4, 17, 24, 45, 52, 78) according to claim 1,
**characterized in that** the first and/or second magnetic elements (1, 1a, 1b, 1c, 1d, 2, 18, 19, 20, 21, 29, 30a, 30b, 35, 36, 58, 59, 60, 61, 82, 83) are magnetizable or permanently magnetic.

3. Measurement device (4, 17, 24, 45, 52, 78) according to claim 1 or 2, **characterized in that** the measurement device (4, 17, 24, 45, 52, 78), in particular the first and/or second magnetic elements (1, 1a, 1b, 1c, 1d, 2, 18, 19, 20, 21, 29, 30a, 30b, 35, 36, 58, 59, 60, 61, 82, 83), is attachable by fastening means (15) to the compression device (42, 63).

4. Measurement device (4, 17, 24, 45, 52, 78) according to claim 3,
**characterized in that** the fastening means (15) comprise a hook-and-loop fastener and/or magnetic fastener.

5. Measurement device (4, 17, 24, 45, 52, 78) according to any of the claims 1 to 4, **characterized in that** the measurement device (4, 17, 24, 45, 52, 78) comprises audible and/or visual and/or electronic and/or sensory indication means indicating the displacement of the at least one first and at least one second magnetic elements (1, 1a, 1b, 1c, 1d, 2, 18, 19, 20, 21, 29, 30a, 30b, 35, 36, 58, 59, 60, 61, 82, 83).

6. Measurement device (4, 17, 24, 45, 52, 78) according to claim 5,
**characterized in that** the indication means are connected directly or indirectly to the measurement device (4, 17, 24, 45, 52, 78) and/ or the compression device (42, 63).

7. Measurement device (4, 17, 24, 45, 52, 78) according to claim 5 or 6, **characterized in that** the visual indication means comprises a scale (88) indicating a range of displacement of the at least one first and/or at least one second magnetic element (1, 1a, 1b, 1c, 1d, 2, 18, 19, 20, 21, 29, 30a, 30b, 35, 36, 58, 59, 60, 61, 82, 83).

8. Measurement device (4, 17, 24, 45, 52, 78) according to one of the preceding claims, **characterized in that** the measurement device (4, 17, 24, 45, 52, 78) comprises at least one constraining element to limit the relative displacement of the at least one first and at least one second magnetic element (1, 1a, 1b, 1c, 1d, 2, 18, 19, 20, 21, 29, 30a, 30b, 35, 36, 58, 59, 60, 61, 82, 83).

9. Measurement device (4, 17, 24, 45, 52, 78) according to claim 8,
**characterized in that** the at least one constraining element is a spacer to limit the relative displacement of the first and second magnetic elements (1, 1a, 1b, 1c, 1d, 2, 18, 19, 20, 21, 29, 30a, 30b, 35, 36, 58, 59, 60, 61, 82, 83) to one another.

10. Measurement device (4, 17, 24, 45, 52, 78) according to claim 8,
**characterized in that** the at least one constraining element is a limiter to restrict the relative displacement of the at least one first and at least one second magnetic element (1, 1a, 1b, 1c, 1d, 2, 18, 19, 20, 21, 29, 30a, 30b, 35, 36, 58, 59, 60, 61, 82, 83) away from each other.

11. Measurement device (4, 17, 24, 45, 52, 78) according to one of the preceding claims, **characterized in that** the measurement device configuration allows relative displacement of the at least one first and at least one second magnetic element (1, 1a, 1b, 1c, 1d, 2, 18, 19, 20, 21, 29, 30a, 30b, 35, 36, 58, 59, 60, 61, 82, 83) by continuous, intermittent direct, and/or intermittent indirect contact.

12. Measurement device (4, 17, 24, 45, 52, 78) according to one of the preceding claims, **characterized in that** the first and/or second magnetic elements (1, 1a, 1b, 1c, 1d, 2, 18, 19, 20, 21, 29, 30a, 30b, 35, 36, 58, 59, 60, 61, 82, 83), the indication means, the fastening means (15), and/or the at least one constraining element are part of and/or protected by and/or affixed to and/or contained in or on a supporting element (7, 8, 27, 28, 84).

13. Measurement device (4, 17, 24, 45, 52, 78) according to one of the preceding claims, **characterized in that** it comprises multiple first and/or second magnetic elements (1, 1a, 1b, 1c, 1d, 2, 18, 19, 20, 21, 29, 30a, 30b, 35, 36, 58, 59, 60, 61, 82, 83), constraining elements, fastening means (15), indication means and/or supporting elements (7, 8, 27, 28, 84), such that a desired magnetic interaction force strength and/or displacement is selectable.

14. Measurement device (4, 17, 24, 45, 52, 78) according to claim 13, **characterized in that** the multiple elements comprise different properties, in particular material composition, geometry, and/or dimension.

15. Measurement device (4, 17, 24, 45, 52, 78) according to claim 13 or 14, **characterized in that** the multiple first and/or second magnetic elements (1, 1a, 1b, 1c, 1d, 2, 18, 19, 20, 21, 29, 30a, 30b, 35, 36, 58, 59, 60, 61, 82, 83) are arranged in series to each other or being optionally combinable with each other such that the pairs of magnetic elements (1, 1a, 1b, 1c, 1d, 2, 18, 19, 20, 21, 29, 30a, 30b, 35, 36, 58, 59, 60, 61, 82, 83) successively indicate the respective defined forces and/or displacements with change of strength of the force applied.

16. Measurement device (4, 17, 24, 45, 52, 78) according to one of the claims 13 to 15, **characterized in that** the multiple elements of the measurement device (4, 17, 24, 45, 52, 78) are being releasably combinable with each other to provide a modular measurement device.

17. Measurement device (4, 17, 24, 45, 52, 78) according to one of the preceding claims, **characterized in that** the measurement device (4, 17, 24, 25) comprises indicia (9, 57, 69, 70, 71), in particular relating to the compression device (42, 63), such that the respective measurement device (4, 17, 24, 25) or measurement device configuration is selectable.

18. Compression device (42, 63), comprising at least one compression material (37, 54, 79) and at least one measurement device (4, 17, 24, 45, 52, 78) according to one of the preceding claims, in particular to adjust the compression device according to a predetermined compression profile of the compression device (42, 63).

19. Compression device (42, 63) according to claim 18, **characterized in that** the measurement device (4, 17, 24, 45, 52, 78), in particular at least one of the magnetic elements (1, 1a, 1b, 1c, 1d, 2, 18, 19, 20, 21, 29, 30a, 30b, 35, 36, 58, 59, 60, 61, 82, 83), is at least partly integrated into the compression device (42, 63), or vice versa.

20. Compression device (42, 63) according to claim 18 or 19, comprising at least one body portion and at least one band (44, 65, 66, 67, 68) for adjusting the compression of the compression device (42, 63), **characterized in that** the measurement device (4, 17, 24, 45, 52, 78) is at least partly integrated into or applied to the at least one body portion and/or the at least one band (44, 65, 66, 67, 68).

21. Compression device (42, 63) according to claims 18 to 20, **characterized in that** multiple measurement devices (4, 17, 24, 45, 52, 78) are associated with different locations of the compression device (42, 63), wherein at least two of them have different measurement configurations, in particular chosen according to a predetermined compression profile of the compression device (42, 63).

22. Compression device (42, 63) according to one of the claims 18 to 21, **characterized in that** it is or comprises a compression wrap and/or a compression sleeve and/or a compression bandage and/or a compression band and/or a compression garment and/or a compression stocking.

23. Method for measuring tension and/or displacement of a compression material (37, 54, 79) of a compression device (42, 63) or force and/or displacement associated with the compression device (42, 63) applied to a patient, using a measurement device (4, 17, 24, 45, 52, 78) according to one of the claims 1 to 17.

24. Method for designing a measurement device (4, 17, 24, 45, 52, 78) or a compression garment (42, 63) with at least one measurement device (4, 17, 24, 45, 52, 78) according to one of the claims 1 to 22 comprising:
- providing at least one compression material (27, 54, 79) wrapable at least partially around a body part to provide compression to the body part,
- determining a tension and/or compression value and/ or compression profile for the compression material (37, 54, 79),
- selecting at least one first and at least one second magnetic element (1, 1a, 1b, 1c, 1d, 2, 18, 19, 20, 21, 29, 30a, 30b, 35, 36, 58, 59, 60, 61, 82, 83) according to a predetermined correlation between magnetic interaction forces and tension or compression values,
- assembling the at least two magnetic elements (1, 1a, 1b, 1c, 1d, 2, 18, 19, 20, 21, 29, 30a, 30b, 35, 36, 58, 59, 60, 61, 82, 83) with the compression material (37, 54, 79) to a measurement device (4, 17, 24, 45, 52, 78) or to a compression garment (42, 63) comprising the measurement device (4, 17, 24, 45, 52, 78).

25. Method for designing a compression garment (45, 52, 78) with at least one measurement device (4, 17, 24, 45, 52, 78) according to claim 24, **characterized in that** the step of selecting the at least two magnetic elements (1, 1a, 1b, 1c, 1d, 2, 18, 19, 20, 21, 29, 30a, 30b, 35, 36, 58, 59, 60, 61, 82, 83) comprises:
- considering the circumference of the body portion covered by the compression material (37, 54, 79) and selecting the at least two magnetic elements (1, 1a, 1b, 1c, 1d, 2, 18, 19, 20, 21, 29, 30a, 30b, 35, 36, 58, 59, 60, 61, 82, 83) according to a predetermined correlation between magnetic interaction forces, circumference of the body part and tension or compression values.

## Patentansprüche

1. Messvorrichtung (4, 17, 24, 45, 52, 78) zum Messen einer Spannung und/oder einer Verschiebung eines Kompressionsmaterials (37, 54, 79) einer Kompressionsvorrichtung (42, 63) oder einer Kompressionskraft, die auf einen Patienten ausgeübt wird, und/oder einer Verschiebung der Kompressionsvorrichtung (42, 63), **dadurch gekennzeichnet, dass** die Messvorrichtung (4, 17, 24, 45, 52, 78) mindestens ein erstes und mindestens ein zweites Magnetelement (1, 1a, 1b, 1c, 1d, 2, 18, 19, 20, 21, 29, 30a, 30b, 35, 36, 58, 59, 60, 61, 82, 83) umfasst, wobei das erste und das zweite Magnetelement (1, 1a, 1b, 1c, 1d, 2, 18, 19, 20, 21, 29, 30a, 30b, 35, 36, 58, 59, 60, 61, 82, 83) derart relativ zueinander verschiebbar sind, dass die Spannung und/oder Kraft abhängig von einer relativen Verschiebung des ersten und des zweiten Magnetelements (1, 1a, 1b, 1c, 1d, 2, 18, 19, 20, 21, 29, 30a, 30b, 35, 36, 58, 59, 60, 61, 82, 83) zueinander, basierend auf einer Spannung und/oder Kraft, die eine magnetische Wechselwirkungskraft zwischen dem ersten und dem zweiten Magnetelement (1, 1a, 1b, 1c, 1d, 2, 18, 19, 20, 21, 29, 30a, 30b, 35, 36, 58, 59, 60, 61, 82, 83) überwindet, messbar ist, und mindestens ein Führungselement zum Führen der relativen Verschiebung des ersten und des zweiten Magnetelements (1, 1a, 1b, 1c, 1d, 2, 18, 19, 20, 21, 29, 30a, 30b, 35, 36, 58, 59, 60, 61, 82, 83).

2. Messvorrichtung (4, 17, 24, 45, 52, 78) nach Anspruch 1, **dadurch gekennzeichnet, dass** das erste und/oder das zweite Magnetelement (1, 1a, 1b, 1c, 1d, 2, 18, 19, 20, 21, 29, 30a, 30b, 35, 36, 58, 59, 60, 61, 82, 83) magnetisierbar oder dauermagnetisch sind.

3. Messvorrichtung (4, 17, 24, 45, 52, 78) nach Anspruch 1 oder 2, **dadurch gekennzeichnet, dass** die Messvorrichtung (4, 17, 24, 45, 52, 78), insbesondere das erste und/oder zweite Magnetelement (1, 1a, 1b, 1c, 1d, 2, 18, 19, 20, 21, 29, 30a, 30b, 35, 36, 58, 59, 60, 61, 82, 83) durch Befestigungsmittel (15) an der Kompressionsvorrichtung (42, 63) anbringbar ist/sind.

4. Messvorrichtung (4, 17, 24, 45, 52, 78) nach Anspruch 3, **dadurch gekennzeichnet, dass** die Befestigungsmittel (15) ein Klettbefestigungselement und/oder ein magnetisches Befestigungselement umfasst.

5. Messvorrichtung (4, 17, 24, 45, 52, 78) nach einem der Ansprüche 1 bis 4, **dadurch gekennzeichnet, dass** die Messvorrichtung (4, 17, 24, 45, 52, 78) hörbare und/oder sichtbare und/oder elektronische und/oder sensorische Anzeigemittel umfasst, welche die Verschiebung des mindestens einen ersten und des mindestens einen zweiten Magnetelements (1, 1a, 1b, 1c, 1d, 2, 18, 19, 20, 21, 29, 30a, 30b, 35, 36, 58, 59, 60, 61, 82, 83) anzeigen.

6. Messvorrichtung (4, 17, 24, 45, 52, 78) nach Anspruch 5, **dadurch gekennzeichnet, dass** die Anzeigemittel direkt oder indirekt mit der Messvorrichtung (4, 17, 24, 45, 52, 78) und/oder der Kompressionsvorrichtung (42, 63) verbunden sind.

7. Messvorrichtung (4, 17, 24, 45, 52, 78) nach Anspruch 5 oder 6, **dadurch gekennzeichnet, dass** das visuelle Anzeigemittel eine Skala (88) umfasst, die einen Bereich der Verschiebung des mindestens einen ersten und/oder des mindestens einen zweiten Magnetelements (1, 1a, 1b, 1c, 1d, 2, 18, 19, 20, 21, 29, 30a, 30b, 35, 36, 58, 59, 60, 61, 82, 83) anzeigt.

8. Messvorrichtung (4, 17, 24, 45, 52, 78) nach einem der vorhergehenden Ansprüche, **dadurch gekennzeichnet, dass** die Messvorrichtung (4, 17, 24, 45, 52, 78) mindestens ein Beschränkungselement umfasst, um die relative Verschiebung des mindestens einen ersten und des mindestens einen zweiten Magnetelements (1, 1a, 1b, 1c, 1d, 2, 18, 19, 20, 21, 29, 30a, 30b, 35, 36, 58, 59, 60, 61, 82, 83) begrenzt.

9. Messvorrichtung (4, 17, 24, 45, 52, 78) nach Anspruch 8, **dadurch gekennzeichnet, dass** das mindestens eine Beschränkungselement ein Abstandshalter ist, um die relative Verschiebung des ersten und des zweiten Magnetelements (1, 1a, 1b, 1c, 1d, 2, 18, 19, 20, 21, 29, 30a, 30b, 35, 36, 58, 59, 60, 61, 82, 83) zueinander zu begrenzen.

10. Messvorrichtung (4, 17, 24, 45, 52, 78) nach Anspruch 8, **dadurch gekennzeichnet, dass** das mindestens eine Beschränkungselement ein Begrenzer ist, um die relative Verschiebung des mindestens einen ersten und des mindestens einen zweiten Magnetelements (1, 1a, 1b, 1c, 1d, 2, 18, 19, 20, 21, 29, 30a, 30b, 35, 36, 58, 59, 60, 61, 82, 83) weg voneinander einzuschränken.

11. Messvorrichtung (4, 17, 24, 45, 52, 78) nach einem der vorhergehenden Ansprüche, **dadurch gekennzeichnet, dass** die Konfiguration der Messvorrichtung die relative Verschiebung des mindestens einen ersten und des mindestens einen zweiten Magnetelements (1, 1a, 1b, 1c, 1d, 2, 18, 19, 20, 21, 29, 30a, 30b, 35, 36, 58, 59, 60, 61, 82, 83) durch kontinuierlichen, zeitweisen direkten und/oder zeitweisen indirekten Kontakt ermöglicht.

12. Messvorrichtung (4, 17, 24, 45, 52, 78) nach einem der vorhergehenden Ansprüche, **dadurch gekennzeichnet, dass** das erste und/oder das zweite Magnetelement (1, 1a, 1b, 1c, 1d, 2, 18, 19, 20, 21, 29, 30a, 30b, 35, 36, 58, 59, 60, 61, 82, 83), die Anzeigemittel, die Befestigungsmittel (15) und/oder das mindestens eine Beschränkungselement Teil eines Stützelements (7, 8, 27, 28, 84) sind und/oder durch selbiges geschützt werden und/oder an selbigem befestigt sind und/oder in selbigem enthalten sind.

13. Messvorrichtung (4, 17, 24, 45, 52, 78) nach einem der vorhergehenden Ansprüche, **dadurch gekennzeichnet, dass** sie mehrere erste und/oder zweite Magnetelemente (1, 1a, 1b, 1c, 1d, 2, 18, 19, 20, 21, 29, 30a, 30b, 35, 36, 58, 59, 60, 61, 82, 83), Beschränkungselemente, Befestigungsmittel (15), Anzeigemittel und/oder Stützelemente (7, 8, 27, 28, 84) umfasst, so dass eine gewünschte Stärke der magnetischen Wechselwirkungskraft und/oder die Verschiebung wählbar ist.

14. Messvorrichtung (4, 17, 24, 45, 52, 78) nach Anspruch 13, **dadurch gekennzeichnet, dass** die mehreren Elemente verschiedene Eigenschaften umfassen, insbesondere hinsichtlich Materialzusammensetzung, Geometrie und/oder Abmessung.

15. Messvorrichtung (4, 17, 24, 45, 52, 78) nach Anspruch 13 oder 14, **dadurch gekennzeichnet, dass** die mehreren ersten und/oder zweiten Magnetelemente (1, 1a, 1b, 1c, 1d, 2, 18, 19, 20, 21, 29, 30a, 30b, 35, 36, 58, 59, 60, 61, 82, 83) nacheinander angeordnet sind oder optional derart miteinander kombinierbar sind, dass die Paare aus Magnetelementen (1, 1a, 1b, 1c, 1d, 2, 18, 19, 20, 21, 29, 30a, 30b, 35, 36, 58, 59, 60, 61, 82, 83) fortlaufend die entsprechenden definierten Kräfte und/oder Verschiebungen mit Veränderungen der Stärke der ausgeübten Kraft anzeigen.

16. Messvorrichtung (4, 17, 24, 45, 52, 78) nach einem der Ansprüche 13 bis 15, **dadurch gekennzeichnet, dass** die mehreren Elemente der Messvorrichtung (4, 17, 24, 45, 52, 78) lösbar miteinander kombinierbar sind, um eine modulare Messvorrichtung bereitzustellen.

17. Messvorrichtung (4, 17, 24, 45, 52, 78) nach einem der vorhergehenden Ansprüche, **dadurch gekennzeichnet, dass** die Messvorrichtung (4, 17, 24, 25) Indizes (9, 57, 69, 70, 71) umfasst, insbesondere bezüglich der Kompressionsvorrichtung (42, 63), so dass die entsprechende Messvorrichtung (4, 17, 24, 25) oder die Konfiguration der Messvorrichtung wählbar ist.

18. Kompressionsvorrichtung (42, 63), mindestens ein Kompressionsmaterial (37, 54, 79) und mindestens eine Messvorrichtung (4, 17, 24, 45, 52, 78) nach einem der vorhergehenden Ansprüche umfassend, insbesondere, um die Kompressionsvorrichtung gemäß einem festgelegten Kompressionsprofil der Kompressionsvorrichtung (42, 63) zu justieren.

19. Kompressionsvorrichtung (42, 63) nach Anspruch 18, **dadurch gekennzeichnet, dass** die Messvorrichtung (4, 17, 24, 45, 52, 78), insbesondere mindestens eines der Magnetelemente (1, 1a, 1b, 1c, 1d, 2, 18, 19, 20, 21, 29, 30a, 30b, 35, 36, 58, 59, 60, 61, 82, 83) mindestens teilweise in die Kompressionsvorrichtung (42, 63) integriert ist oder umgekehrt.

20. Kompressionsvorrichtung (42, 63) nach Anspruch 18 oder 19, mindestens einen Körperabschnitt und mindestens ein Band (44, 65, 66, 67, 68) zum Justieren der Kompression der Kompressionsvorrichtung (42, 63) umfassend, **dadurch gekennzeichnet, dass** die Messvorrichtung (4, 17, 24, 45, 52, 78) mindestens teilweise in den mindestens einen Körperabschnitt und/oder das mindestens eine Band (44, 65, 66, 67, 68) integriert ist oder darauf aufgebracht ist.

21. Kompressionsvorrichtung (42, 63) nach einem der Ansprüche 18 bis 20, **dadurch gekennzeichnet, dass** mehrere Messvorrichtungen (4, 17, 24, 45, 52, 78) mit verschiedenen Positionen der Kompressionsvorrichtung (42, 63) in Verbindung stehen, wobei mindestens zwei von ihnen verschiedene Messkonfigurationen aufweisen, die insbesondere gemäß einem festgelegten Kompressionsprofil der Kompressionsvorrichtung (42, 63) gewählt wurden.

22. Kompressionsvorrichtung (42, 63) nach einem der Ansprüche 18 bis 21, **dadurch gekennzeichnet, dass** sie ein Kompressionswickel und/oder eine Kompressionshülse und/oder einer Kompressionsbandage und/oder ein Kompressionsband und/oder ein Kompressionskleidungstück und/oder ein Kompressionsstrumpf ist oder diese umfasst.

23. Verfahren zum Messen der Spannung und/oder Verschiebung eines Kompressionsmaterials (37, 54, 79) einer Kompressionsvorrichtung (42, 63) oder einer Kraft und/oder Verschiebung, die mit der an einen Patienten angelegten Kompressionsvorrichtung (42, 63) in Verbindung stehen, mit Hilfe einer Messvorrichtung (4, 17, 24, 45, 52, 78) nach einem der Ansprüche 1 bis 17.

24. Verfahren zum Gestalten einer Messvorrichtung (4, 17, 24, 45, 52, 78) oder eines Kompressionskleidungsstücks (42, 63) mit mindestens einer Messvorrichtung (4, 17, 24, 45, 52, 78) nach einem der Ansprüche 1 bis 22, Folgendes umfassend:
- Bereitstellen mindestens eines Kompressionsmaterials (37, 54, 79), das mindestens teilweise um einen Körperteil wickelbar ist, um Kompression für den Körperteil bereitzustellen,
- Bestimmen eines Spannungs- und/oder Kompressionswertes und/oder Kompressionsprofils für das Kompressionsmaterial (37, 54, 79),
- Auswählen mindestens eines ersten und mindestens eines zweiten Magnetelements (1, 1a, 1b, 1c, 1d, 2, 18, 19, 20, 21, 29, 30a, 30b, 35, 36, 58, 59, 60, 61, 82, 83) gemäß einer festgelegten Korrelation zwischen magnetischen Wechselwirkungskräften und Spannungs- oder Kompressionswerten,
- Zusammenfügen der mindestens zwei Magnetelemente (1, 1a, 1b, 1c, 1d, 2, 18, 19, 20, 21, 29, 30a, 30b, 35, 36, 58, 59, 60, 61, 82, 83) und des Kompressionsmaterials (37, 54, 79) zu einer Messvorrichtung (4, 17, 24, 45, 52, 78) oder zu einem Kompressionskleidungsstück (42, 63), das die Messvorrichtung (4, 17, 24, 45, 52, 78) umfasst.

25. Verfahren zum Gestalten eines Kompressionskleidungsstücks (45, 52, 78) mit mindestens einer Messvorrichtung (4, 17, 24, 45, 52, 78) nach Anspruch 24, **dadurch gekennzeichnet, dass** der Schritt des Auswählens der mindestens zwei Magnetelemente (1, 1a, 1b, 1c, 1d, 2, 18, 19, 20, 21, 29, 30a, 30b, 35, 36, 58, 59, 60, 61, 82, 83) Folgendes umfasst:
- Berücksichtigen des Umfangs des Körperabschnitts, der von dem Kompressionsmaterial (37, 54, 79) bedeckt wird, und Auswählen der mindestens zwei Magnetelemente (1, 1a, 1b, 1c, 1d, 2, 18, 19, 20, 21, 29, 30a, 30b, 35, 36, 58, 59, 60, 61, 82, 83) gemäß einer festgelegten Korrelation zwischen magnetischen Wechselwirkungskräften, dem Umfang des Körperteils und Spannungs- oder Kompressionswerten.

## Revendications

1. Dispositif de mesure (4, 17, 24, 45, 52, 78) pour mesurer une tension et/ou un déplacement d'un matériau de compression (37, 54, 79) d'un dispositif de compression (42, 63) ou une force de compression appliquée sur un patient et/ou un déplacement du dispositif de compression (42, 63), **caractérisé en ce que** le dispositif de mesure (4, 17, 24, 45, 52, 78) comprend au moins un premier et au moins un second élément magnétique (1, 1a, 1b, 1c, 1d, 2, 18, 19, 20, 21, 29, 30a, 30b, 35, 36, 58, 59, 60, 61, 82, 83), dans lequel le premier et le second éléments magnétiques (1, 1a, 1b, 1c, 1d, 2, 18, 19, 20, 21, 29, 30a, 30b, 35, 36, 58, 59, 60, 61, 82, 83) sont déplaçables l'un relativement à l'autre de telle sorte que la tension et/ou la force soient mesurables en fonction d'un déplacement relatif des premier et second éléments magnétiques (1, 1a, 1b, 1c, 1d, 2, 18, 19, 20, 21,29, 30a, 30b, 35, 36, 58, 59, 60, 61, 82, 83) l'un par rapport à l'autre, sur la base d'une tension et/ou d'une force surpassant une force d'interaction magnétique entre les premier et second éléments magnétiques (1, 1a, 1b, 1c, 1d, 2, 18, 19, 20, 21, 29, 30a, 30b, 35, 36, 58, 59, 60, 61, 82, 83), et au moins un élément de guidage pour guider le déplacement relatif des premier et second éléments magnétiques (1, 1a, 1b, 1c, 1d, 2, 18, 19, 20, 21, 29, 30a, 30b, 35, 36, 58, 59, 60, 61, 82, 83).

2. Dispositif de mesure (4, 17, 24, 45, 52, 78) selon la revendication 1, **caractérisé en ce que** le premier et/ou le second éléments magnétiques (1, 1a, 1b, 1c, 1d, 2, 18, 19, 20, 21, 29, 30a, 30b, 35, 36, 58, 59, 60, 61, 82, 83) sont magnétisables ou magnétiques en permanence.

3. Dispositif de mesure (4, 17, 24, 45, 52, 78) selon la revendication 1 ou 2, **caractérisé en ce que** le dispositif de mesure (4, 17, 24, 45, 52, 78), en particulier le premier et/ou le second éléments magnétiques (1, 1a, 1b, 1c, 1d, 2, 18, 19, 20, 21, 29, 30a, 30b, 35, 36, 58, 59, 60, 61, 82, 83), est attachable par des moyens de fixation (15) au dispositif de compression (42, 63).

4. Dispositif de mesure (4, 17, 24, 45, 52, 78) selon la revendication 3, **caractérisé en ce que** les moyens de fixation (15) comprennent un organe de fixation autoagrippante et/ou organe de fixation magnétique.

5. Dispositif de mesure (4, 17, 24, 45, 52, 78) selon l'une quelconque des revendications 1 à 4, **caractérisé en ce que** le dispositif de mesure (4, 17, 24, 45, 52, 78) comprend des moyens d'indication audibles et/ou visuels et/ou électroniques et/ou sensoriels indiquant le déplacement de l'au moins un premier et de l'au moins un second éléments magnétiques (1, 1a, 1b, 1c, 1d, 2, 18, 19, 20, 21, 29, 30a, 30b, 35, 36, 58, 59, 60, 61, 82, 83) .

6. Dispositif de mesure (4, 17, 24, 45, 52, 78) selon la revendication 5, **caractérisé en ce que** les moyens d'indication sont connectés directement ou indirectement au dispositif de mesure (4, 17, 24, 45, 52, 78) et/ou au dispositif de compression (42, 63).

7. Dispositif de mesure (4, 17, 24, 45, 52, 78) selon la revendication 5 ou 6, **caractérisé en ce que** les moyens d'indication visuels comprennent une échelle (88) indiquant une plage de déplacement de l'au moins un premier et/ou de l'au moins un second élément magnétique (1, 1a, 1b, 1c, 1d, 2, 18, 19, 20, 21, 29, 30a, 30b, 35, 36, 58, 59, 60, 61, 82, 83).

8. Dispositif de mesure (4, 17, 24, 45, 52, 78) selon l'une des revendications précédentes, **caractérisé en ce que** le dispositif de mesure (4, 17, 24, 45, 52, 78) comprend au moins un élément de restriction pour limiter le déplacement relatif de l'au moins un premier et de l'au moins un second élément magnétique (1, 1a, 1b, 1c, 1d,2, 18, 19, 20, 21, 29, 30a, 30b, 35, 36, 58, 59, 60, 61, 82, 83).

9. Dispositif de mesure (4, 17, 24, 45, 52, 78) selon la revendication 8, **caractérisé en ce que** l'au moins un élément de restriction est une pièce d'espacement pour limiter le déplacement relatif des premier et second éléments magnétiques (1, 1a, 1b, 1c, 1d, 2, 18, 19, 20, 21, 29, 30a, 30b, 35, 36, 58, 59, 60, 61, 82, 83) l'un par rapport à l'autre.

10. Dispositif de mesure (4, 17, 24, 45, 52, 78) selon la revendication 8, **caractérisé en ce que** l'au moins un élément de restriction est un limiteur pour restreindre le déplacement relatif de l'au moins un premier et de l'au moins un second élément magnétique (1, 1a, 1b, 1c, 1d, 2, 18, 19, 20, 21, 29, 30a, 30b, 35, 36, 58, 59, 60, 61, 82, 83) à l'opposé l'un de l'autre.

11. Dispositif de mesure (4, 17, 24, 45, 52, 78) selon l'une des revendications précédentes, **caractérisé en ce que** la configuration de dispositif de mesure permet le déplacement relatif de l'au moins un premier et de l'au moins un second élément magnétique (1, 1a, 1b, 1c, 1d, 2, 18, 19, 20, 21, 29, 30a, 30b, 35, 36, 58, 59, 60, 61, 82, 83) par contact continu, direct intermittent, et/ou indirect intermittent.

12. Dispositif de mesure (4, 17, 24, 45, 52, 78) selon l'une des revendications précédentes, **caractérisé en ce que** le premier et/ou le second éléments magnétiques (1, 1a, 1b, 1c, 1d, 2, 18, 19, 20, 21, 29, 30a, 30b, 35, 36, 58, 59, 60, 61, 82, 83), les moyens d'indication, les moyens de fixation (15), et/ou l'au moins un élément de restriction font partie de et/ou sont protégés par et/ou apposés sur et/ou contenus dans ou sur un élément de support (7, 8, 27, 28, 84).

13. Dispositif de mesure (4, 17, 24, 45, 52, 78) selon l'une des revendications précédentes, **caractérisé en ce qu'**il comprend de multiples premiers et/ou seconds éléments magnétiques (1, 1a, 1b, 1c, 1d, 2, 18, 19, 20, 21, 29, 30a, 30b, 35, 36, 58, 59, 60, 61, 82, 83), éléments de restriction, moyens de fixation (15), moyens d'indication et/ou éléments de support (7, 8, 27, 28, 84), de telle sorte qu'une intensité de force d'interaction magnétique et/ou un déplacement souhaités soient sélectionnables.

14. Dispositif de mesure (4, 17, 24, 45, 52, 78) selon la revendication 13, **caractérisé en ce que** les multiples éléments comprennent différentes propriétés, en particulier composition de matériau, géométrie, et/ou dimension.

15. Dispositif de mesure (4, 17, 24, 45, 52, 78) selon la revendication 13 ou 14, **caractérisé en ce que** les multiples premiers et/ou seconds éléments magnétiques (1, 1a, 1b, 1c, 1d, 2, 18, 19, 20, 21,29, 30a, 30b, 35, 36, 58, 59, 60, 61, 82, 83) sont agencés en série les uns avec les autres ou étant facultativement combinables les uns aux autres de telle sorte que les paires d'éléments magnétiques (1, 1a, 1b, 1c, 1d, 2, 18, 19, 20, 21, 29, 30a, 30b, 35, 36, 58, 59, 60, 61, 82, 83) indiquent successivement les forces et/ou déplacements définis respectifs avec un changement d'intensité de la force appliquée.

16. Dispositif de mesure (4, 17, 24, 45, 52, 78) selon l'une des revendications 13 à 15, **caractérisé en ce que** les multiples éléments du dispositif de mesure (4, 17, 24, 45, 52, 78) sont combinables de façon libérable les uns aux autres pour fournir un dispositif de mesure modulaire.

17. Dispositif de mesure (4, 17, 24, 45, 52, 78) selon l'une des revendications précédentes, **caractérisé en ce que** le dispositif de mesure (4, 17, 24, 25) comprend des indications (9, 57, 69, 70, 71), en particulier concernant le dispositif de compression (42, 63), de telle sorte que le dispositif de mesure (4, 17, 24, 25) respectif ou la configuration de dispositif de mesure respective soit sélectionnable.

18. Dispositif de compression (42, 63), comprenant au moins un matériau de compression (37, 54, 79) et au moins un dispositif de mesure (4, 17, 24, 45, 52, 78) selon l'une des revendications précédentes, en particulier pour ajuster le dispositif de compression selon un profil de compression prédéterminé du dispositif de compression (42, 63).

19. Dispositif de compression (42, 63) selon la revendication 18, **caractérisé en ce que** le dispositif de mesure (4, 17, 24, 45, 52, 78), en particulier au moins un des éléments magnétiques (1, 1a, 1b, 1c, 1d, 2, 18, 19, 20, 21, 29, 30a, 30b, 35, 36, 58, 59, 60, 61, 82, 83), est au moins partiellement intégré dans le dispositif de compression (42, 63), ou vice versa.

20. Dispositif de compression (42, 63) selon la revendication 18 ou 19, comprenant au moins une portion corporelle et au moins une sangle (44, 65, 66, 67, 68) pour ajuster la compression du dispositif de compression (42, 63), **caractérisé en ce que** le dispositif de mesure (4, 17, 24, 45, 52, 78) est au moins partiellement intégré dans ou appliqué sur l'au moins une portion corporelle et/ou l'au moins une bande (44, 65, 66, 67, 68) .

21. Dispositif de compression (42, 63) selon les revendications 18 à 20, **caractérisé en ce que** de multiples dispositifs de mesure (4, 17, 24, 45, 52, 78) sont associés à différents emplacements du dispositif de compression (42, 63), dans lequel au moins deux de ceuxci ont différentes configurations de mesure, en particulier choisies selon un profil de compression prédéterminé du dispositif de compression (42, 63).

22. Dispositif de compression (42, 63) selon l'une des revendications 18 à 21, **caractérisé en ce qu'**il est ou comprend une bande de compression et/ou un manchon de compression et/ou un bandage de compression et/ou une sangle de compression et/ou un vêtement de compression et/ou un bas de compression.

23. Procédé pour mesurer une tension et/ou un déplacement d'un matériau de compression (37, 54, 79) d'un dispositif de compression (42, 63) ou une force et/ou un déplacement associés au dispositif de compression (42, 63) appliqué sur un patient, en utilisant un dispositif de mesure (4, 17, 24, 45, 52, 78) selon l'une des revendications 1 à 17.

24. Procédé pour concevoir un dispositif de mesure (4, 17, 24, 45, 52, 78) ou un vêtement de compression (42, 63) avec au moins un dispositif de mesure (4, 17, 24, 45, 52, 78) selon l'une des revendications 1 à 22, comprenant :
- la fourniture d'au moins un matériau de compression (27, 54, 79) enroulable au moins partiellement autour d'une partie corporelle pour fournir une compression à la partie corporelle,
- la détermination d'une valeur de tension et/ou de compression et/ou un profil de compression pour le matériau de compression (37, 54, 79),
- la sélection d'au moins un premier et d'au moins un second élément magnétique (1, 1a, 1b, 1c, 1d, 2, 18, 19, 20, 21,29, 30a, 30b, 35, 36, 58, 59, 60, 61, 82, 83) selon une corrélation prédéterminée entre des forces d'interaction magnétiques et des valeurs de tension ou de compression,
- l'assemblage des au moins deux éléments magnétiques (1, 1a, 1b, 1c, 1d, 2, 18, 19, 20, 21, 29, 30a, 30b, 35, 36, 58, 59, 60, 61, 82, 83), avec le matériau de compression (37, 54, 79), à un dispositif de mesure (4, 17, 24, 45, 52, 78) ou à un vêtement de compression (42, 63) comprenant le dispositif de mesure (4, 17, 24, 45, 52, 78).

25. Procédé pour concevoir un vêtement de compression (45, 52, 78) avec au moins un dispositif de mesure (4, 17, 24, 45, 52, 78) selon la revendication 24, **caractérisé en ce que** l'étape de la sélection des au moins deux éléments magnétiques (1, 1a, 1b, 1c, 1d, 2, 18, 19, 20, 21, 29, 30a, 30b, 35, 36, 58, 59, 60, 61, 82, 83) comprend :
- la considération de la circonférence de la portion corporelle couverte par le matériau de compression (37, 54, 79) et la sélection des au moins deux éléments magnétiques (1, 1a, 1b, 1c, 1d,2, 18, 19, 20, 21, 29, 30a, 30b, 35, 36, 58, 59, 60, 61, 82, 83) selon une corrélation prédéterminée entre des forces d'interaction magnétiques, la circonférence de la partie corporelle et des valeurs de tension ou de compression.
